# EUROPEAN PATENT APPLICATION

(11) **EP 4 635 433 A2**
(43) Date of publication of application: **22.10.2025**
(21) Application number: 25200672.1
(22) Date of filing: 01.03.2022
(51) Int. Cl.: A61B 17/70

(54) **ORTHOPEDIC SURGERY USING AUGMENTED OR MIXED REALITY ASSISTANCE**

(30) Priority: 01.03.2021 WO PCT/IB2021/051694; 12.07.2021 WO PCT/IB2021/056242
(62) Divisional of application: 22717426.5
(71) Applicant: Neo Medical SA, 1096 Villette (CH)
(72) Inventor: LEFAUCONNIER, Vincent, 1817 Brent (CH)
(74) Representative: Byrne, Declan

(57) **Abstract**

A method for assisting an orthopedic surgery, the method including the steps of: capturing a sequence of images such that a field of view captures images of a plurality of screw extenders, each screw extender holding a pedicle screw, the plurality of screw extenders arranged at a surgical incision of an orthopedic surgery, displaying images of the captured images to provide for a live video feed, detecting the plurality of screw extenders based on the captured sequence of images, first calculating an orientation and position of the detected plurality of screw extenders, second calculating a 3D position of a screw head of each pedicle screw based on the orientation and the position, and projecting and displaying each calculated 3D position of the plurality of screw heads with a graphical element on the display device at a location that corresponds to the location of the screw head projected to a currently displayed image of the live video feed.

## Description

### CROSS-REFERNCE TO RELATED APPLICATIONS

The present invention claims priority to and fully incorporates by reference International Patent Applications with the Serial Nos. PCT/IB2021/051694 that was filed on March 1, 2021 and PCT/IB2021/056242 that was filed on July 12, 2021.

### FIELD OF THE INVENTION

The present invention relates to the field of orthopedic surgery using augmented or mixed reality, more particularly, to a method, system, and device for using augmented or mixed reality to provide for assistance or facilitation to a surgeon performing orthopedic surgery, specifically for proposing different types and shapes of stabilization rods for spinal fusion surgery, and other types of orthopedic surgery.

### BACKGROUND

In the field of orthopedics and implant tools and systems for orthopedic surgery, more specifically spinal fusion surgery for a spinal column, a pedicle screw is used to attach to vertebra with a bone anchor through an incision location on the back of the patient. After several pedicle screws are attached to different vertebrae, the heads of these pedicle screws are connected together via rod-type or bar-type device, and the rod-type or bar-type device, also called spinal rod, is attached to the head of the pedicle screws with a set screw. As an example, for several adjacent vertebrae for vertebrae fusion, for each vertebra, a pedicle screw is screwably attached thereto with the bone anchor of the pedicle screw, and thereafter, these pedicle screws are mechanically fastened towards each other by the use of the spinal rod that is placed in a groove or U-shaped opening that is formed by the pedicle screw head, forming a row of pedicle screws along the spine. This allows to provide for the mechanical support needed for spinal stabilization for spinal fusion in a patient or living being.

To better reach into the incision location and screwably attach a pedicle screw to the vertebrae, the pedicle screw, specifically the head of the pedicle screw, is usually removably attached to a screw extender, or a similar device, for example an extended tap screw head or bladed. The screw extender and the similar devices have the purpose to add additional length to the head of the pedicle screw allowing the operator or surgeon to act outside of the surgical incision, to keep the surgical incision open, but also to help guiding different tools and the spinal rod to the head of the pedicle screw. The screw extender that is configured to hold the pedicle screw is usually a tubular, longitudinal device that is quite a bit larger than the head of the pedicle screw, and itself has a longitudinally-shaped slot along a side thereof. When the pedicle screw head is connected to the screw extender, the longitudinally-shaped slot matches with the U-shaped opening in the screw head of the pedicle screw, and therefore allows to guide the spinal rod into the U-shaped opening through the longitudinally-shaped slot. The process of pushing down the spinal rod within the longitudinally shaped slot of the screw extender towards and into the head of pedicle screw is also called rod reduction.

For example, U.S. Patent No. 10,058,355, this reference herewith incorporated by reference in its entirety, describes an orthopedic implant kit that provides for a pedicle screw, a corresponding set screw, a rod, and the tools to operate these, including a screw extender for holding the pedicle screw, and a set screw driver for threadably tightening the set screw relative to screw head of the pedicle screw. U.S. Patent No. 7,160,300, this reference herewith incorporated by reference in its entirety, describes a rod reduction method where intermediate guide tools are attached to bone screws, the intermediate guide tools having a tubular shape with a longitudinally-shaped channel that can guide a rod from the guide tools to the bone screw attached thereto. As another example, U.S. Patent No. 8,795,283, this reference herewith incorporated by reference in its entirety, describes another type of kit orthopedic surgery system for surgical intervention for spinal stabilization, including pedicle screw with a head for receiving a rod, and tools necessary for the surgical intervention. The screw extender is made of a tube having two separable half-shells that are held together by a holding ring so that a tubular shape can be formed. In yet another example, U.S. Patent No. 8,262,662, this reference herewith incorporated by reference in its entirety, provides for a system and method for delivering a spinal connector spinal anchor sites in a spinal column. In one embodiment, a spinal implant and access device is provided that includes a U-shaped receiver member, a bone-engaging member, an extension member, a spinal rod, and a set screw. The extension member has a tubular shape.

Similar orthopedic spinal surgery concepts, tools and devices have been proposed as discussed above, for attaching a rod to a pedicle screw via a set screw, for example U.S. Patent No. 5,129,388, U.S. Patent No. 5,520,689, U.S. Patent No. 5,536,268, U.S. Patent No. 5,720,751, U.S. Patent No. 5,984,923, U.S. Patent No. 6,056,753, U.S. Patent No. 6,183,472, U.S. Patent No. 6,258,090, U.S. Patent No. 6,454,768, U.S. Patent No. 6,648,888, U.S. Patent No. 6,740,086, U.S. Patent No. 7,618,442, U.S. Patent No. 8,308,782, U.S. Patent No. 8,876,868, U.S. Patent Publication No. 2006/0025771, and U.S. Patent Publication No. 2018/0289397, all of these references herewith incorporated by reference in its entirety.

However, once the pedicle screws are attached to vertebrae of a spine, only the screw extenders that are removably attached to the screw heads of respective pedicle screws can be seen by the surgeon or operator, the screw extenders generally pointing out and away from a surgical incision that was needed to attach pedicle screws to the vertebrae. Generally, unless the surgeon opens up the incision, the screw heads are embedded in the surrounding tissue of the incision. In this respect, before the rod reduction and rod fixation process, a surgeon or operator usually needs to choose a rod having an appropriate length, pre-bend a spinal rod, or choose a pre-bend spinal rod for placement into the U-shaped grooves of the heads of the pedicle screws. However, without being able to see an exact placement of the pedicle screws and their screw heads with their groove for accommodating the spinal rod, this is a difficult task that can lead to a trial and error procedure for determining an appropriate length and shape and bending of a spinal rod, such that it can be inserted percutaneously in each screw head of the pedicle screws. This can lead to a substantial loss of time during the surgery, an increased risk of screw loosening or implant failure, and additional costs.

Solutions have been proposed in orthopedic surgeries to detect the visible pedicle screw heads based on machine learning with a convolutional neural network (CNN). See for example Von Atzigen et al., "HoloYolo: A proof-of-concept study for marker-less surgical navigation of spinal rod implants with augmented reality and on-device machine learning" The International Journal of Medical Robotics and Computer Assisted Surgery, year 2020, e2184. However, this method suffers from many drawbacks as it relies on the direct visual view of the different screw heads of the pedicle screws that are attached to the vertebrae and therefore requires a fully open surgical location and maximal opening of the incision with direct view into the wound, and necessitates a relatively long data processing time for detection and slow tracking refresh rate, and having substantial detection uncertainties.

To avoid the drawbacks of imaging solutions that are camera-view based, some methods have used C-arm fluoroscopy with X-ray projections to evaluate pedicle screw placements, allowing to calculate a pose estimation of screws based on biplanar X-rays and fluoroscopic images using reflective markers. See Esfandiari et al., "A deep learning framework for segmentation and pose estimation of pedicle screw implants based on C-arm fluoroscopy," International journal of computer assisted radiology and surgery, Vol. 13, No. 8, year 2018, pp. 1269-1282, see also Fu et al., "Computer-Assisted Fluoroscopic Navigation of Pedicle Screw Insertion An In Vivo Feasibility Study," Acta Orthopaedica Scandinavica, Vol. 75, No. 6, year 2004, pp. 730-735. However, these methodologies require complex and costly computer tomography equipment, and due to the additional operational steps that need to be performed, are also not suitable for direct use of a orthopedic surgeon.

Therefore, there is need for a system, method, and device to improve the use of spinal rods during surgery, specifically the placement, implantation, preselection and matching of spinal rods for a specific surgical conditions, having a simplified use for the user, and requiring substantially less cost for assisting the user.

### SUMMARY

According to one aspect of the present invention, a method for assisting an orthopedic surgery is provided. The method can be performed with a data processing device, the data processing device including a display device and an image capturing device. Preferably, the method comprises the steps of capturing a sequence of images with the imaging device such that a field of view of the imaging device captures images of a plurality of screw extenders, each screw extender holding a pedicle screw, the plurality of screw extenders arranged at a surgical incision of a body of a living being undergoing orthopedic surgery, displaying at least some of images of the captured images to provide for a live video feed on the display device, detecting the plurality of screw extenders with the data processing device based on the captured sequence of images, first calculating an orientation and position of the detected plurality of screw extenders, second calculating a three-dimensional (3D) position of a screw head of each pedicle screw based on the orientation and the position of the first calculating; and projecting and displaying each calculated 3D position of the plurality of screw heads with a graphical element with a graphical user interface on the display device at a location that corresponds to the location of the screw head projected to a currently displayed image of the live video feed.

According to another aspect of the present invention, a non-transitory computer-readable medium is provided, having computer instructions recorded thereon. The computer instructions are configured to perform a method for assisting an orthopedic surgery when executed on a computer device, the computer device being in operative connection with a display device and an image capturing device.

According to still another aspect of the present invention, a computer system is provided, the computer system including an image capturing device, a display device, and data processing device, the data processing device being in operative connection with the image capturing device and the display device. Preferably, the data processing device configured to perform a method for assisting an orthopedic surgery using augmented reality.

According to another aspect of the present invention, a method for assisting orthopedic surgery to determine a correction of a spinal column based on a bent fixation rod is provided. Preferably the method is performed with a data processing device. Moreover, preferably, the method includes the steps of scanning a fixation rod with an image capturing device to obtained scanned data of the fixation rod, the spinal correction rod having been bend for a spinal correction, first calculating a curvature data of the fixation rod based on the scanned data, receiving data of locations of attachment points for the fixation rod to the spinal column, the locations of the attachment points having been determined based on positional data of screw heads of pedicle screws that are attached to vertebrae of the spinal column, second calculating data of corrected locations of the attachment points, the corrected locations of the attachment points being based on a correction that is imparted to the locations of the attachment points when the fixation rod were to be attached to the attachment points of a corrected spinal column, by taking account the curvature data of the fixation rod from the step of first calculating, third calculating a spinal parameter of the corrected spinal column based on the data of the corrected locations of the attachment points of the corrected spinal column, and displaying the spinal parameter of the corrected spinal column on a display device.

According to yet another aspect of the present invention, a non-transitory computer-readable medium is provided, having computer instructions recorded thereon. The computer instructions are configured to perform a method for assisting orthopedic surgery to determine a correction of a spinal column based on a bent fixation rod is provided, when executed by a computer device that is in operative connection with a display device and an image capturing device.

According to still another aspect of the present invention, a computer system is provided, the computer system including an image capturing device, a display device, and data processing device, the data processing device being in operative connection with the image capturing device and the display device. Preferably, the data processing device configured to perform a method for assisting orthopedic surgery to determine a correction of a spinal column based on a bent fixation rod.

According to another aspect of the present invention, a method for assisting orthopedic surgery to a spinal column is provided. Preferably, the method is performed with a data processing device, the data processing device including a display device and an image capturing device. Moreover, preferably, the method comprising the steps of capturing a sequence of images with the image capturing device such that a field of view of the image capturing device captures images of at least one of a plurality of pedicle markers placed on a plurality of guide wires, respectively, or a plurality of guide wires, the plurality of pedicle markers or the plurality of guide wires arranged at a surgical incision of a body of a living being undergoing orthopedic surgery, providing for a live video feed on the display device by either displaying at least some of images of the captured images or by a direct view with a transparent display device, detecting the plurality of pedicle markers or the plurality of guide wires with the data processing device based on the captured sequence of images, first calculating an orientation and position of the detected plurality of pedicle markers or the detected plurality of guide wires, and second calculating pose data information for at least two vertebrae based on the orientation and the position of at least one of the detected plurality of pedicle markers or the detected plurality of guide wires that are attached to the vertebra from the step of first calculating.

The above and other objects, features and advantages of the present invention and the manner of realizing them will become more apparent, and the invention itself will best be understood from a study of the following description and appended claims with reference to the attached drawings showing some preferred embodiments of the invention.

### BRIEF DESCRIPTION OF THE SEVERAL DRAWINGS

The accompanying drawings, which are incorporated herein and constitute part of this specification, illustrate the presently preferred embodiments of the invention, and together with the general description given above and the detailed description given below, serve to explain features of the invention.

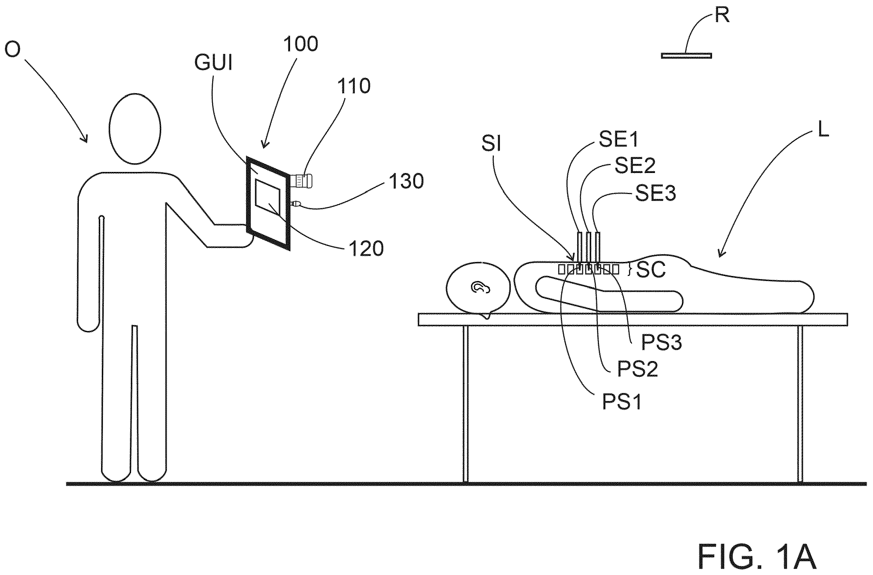

FIG. 1A shows a perspective view of an exemplary and simplified location or premises where the orthopedic surgery is performed, showing living being or patient L on a surgical table having a surgical incision SI, and user, operator, surgeon, medical assistant O holding an exemplary data processing device 100 for performing the method for proposing different types of spinal rods for an orthopedic surgery;

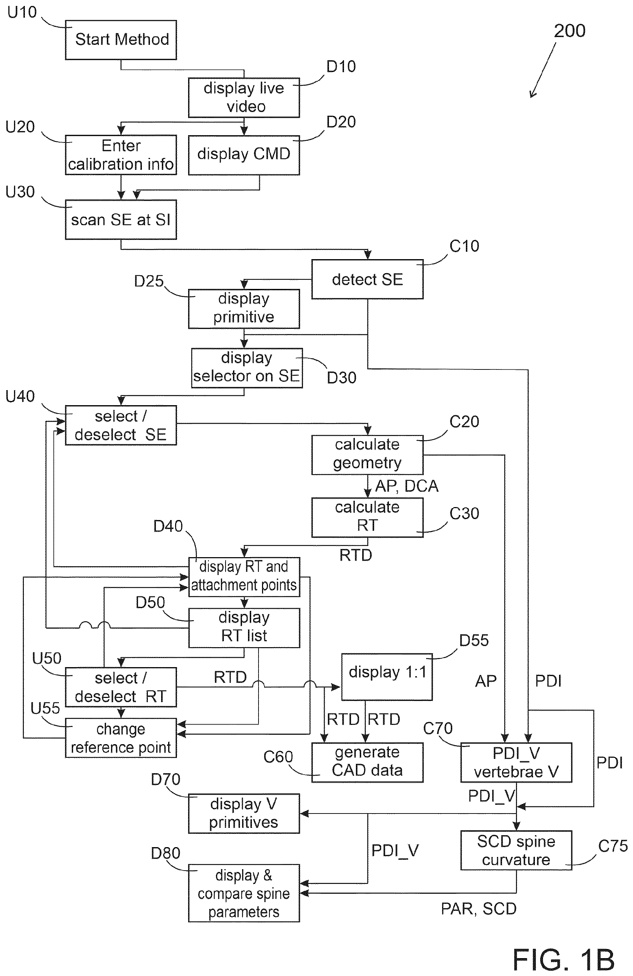

FIG. 1B shows an exemplary and simplified flow chart depicting the different steps of the method for proposing different types of spinal rods for an orthopedic surgery by using augmented reality, according to an aspect of the present invention; and

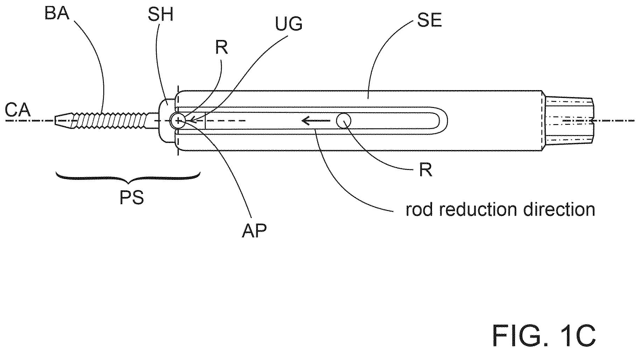

FIG. 1C shows a side view of an exemplary screw extender SE and pedicle screw assembly with a bone anchor BA and a screw head SH, to visualize different elements of this exemplary assembly, as a non-limiting example of a screw extender and pedicle screw for the herein present methods and systems;

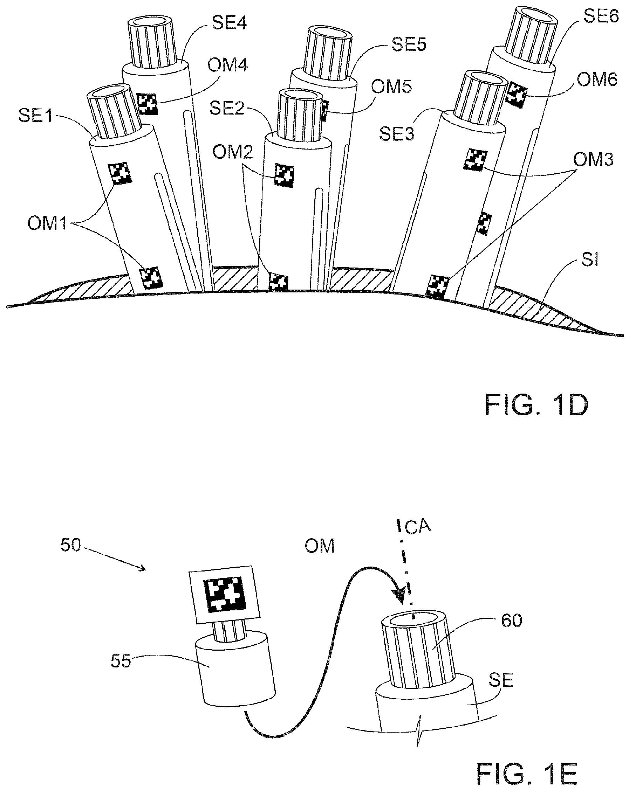

FIG. 1D shows a simplified schematic perspective view of a surgical incision SI having an exemplary number of six (6) screw extenders SE1-SE6 protruding therefrom, with each screw extender SE equipped with optical markers OM1-OM6 for detecting and tracking the screw extenders;
FIG. 1E shows a simplified perspective view of an exemplary marker device 50 that can be removably placed onto a distal end 60 of a screw extender SE, the marker device 50 having an optical marker code OM and an attachment device 55 for removable attachment to a screw extender SE;

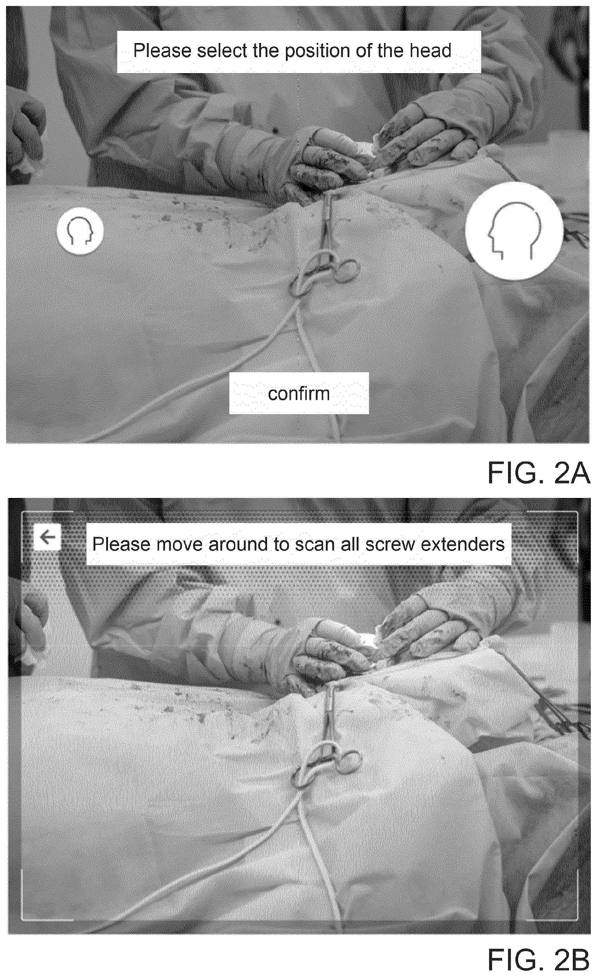

FIGs. 2A to 2N show exemplary screenshots of different stages of the method, the screenshots preferably being displayed on a graphical user interface of a data processing device, showing different aspects of the augmented reality that is used on a display screen for orthopedic surgery assistance; and

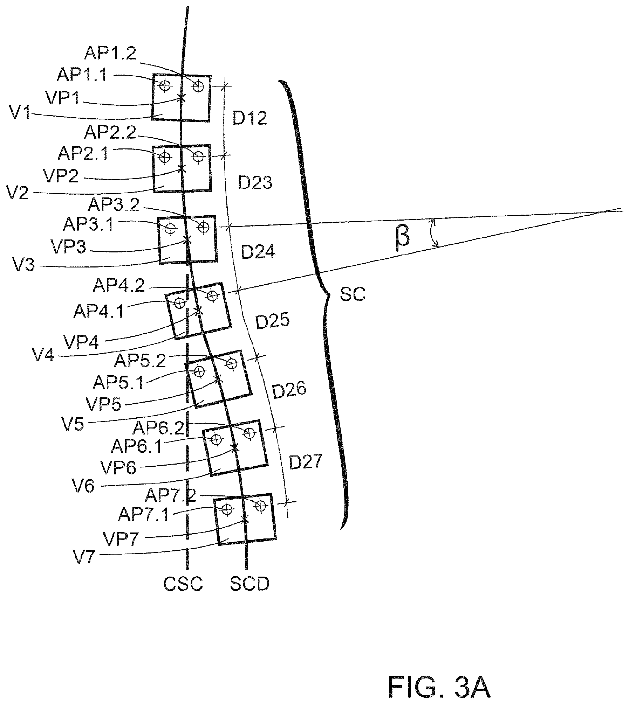

FIG. 3A shows a schematic and simplified representation of a spine or vertebral column SC with seven (7) exemplary vertebrae V1-V7, illustrated from a back view, for which for each vertebrae V, two attachment points AP have been determined with the herein described method, and visualizing different parameters that can be calculated and displayed by some steps to the method, for example steps C70, C75, D70, D80, including position and pose information PDI_V of each vertebra V1-V7, curvature data SCD of the currently uncorrected spine or vertebral column, and data on corrected spine curve CSC, for a corrected spine or vertebral column;

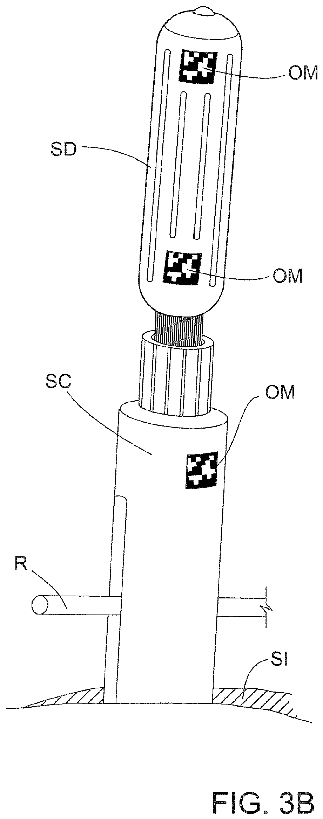

FIG. 3B shows an exemplary and perspective view of a screw extender SE that is placed inside a surgical incision SI, having a tool SD attached thereto, the tool SD for example being a screw driver SD for attaching pedicle screws to a vertebrae V, tool SD having two exemplary (2) optical detection markers OM arranged thereon;

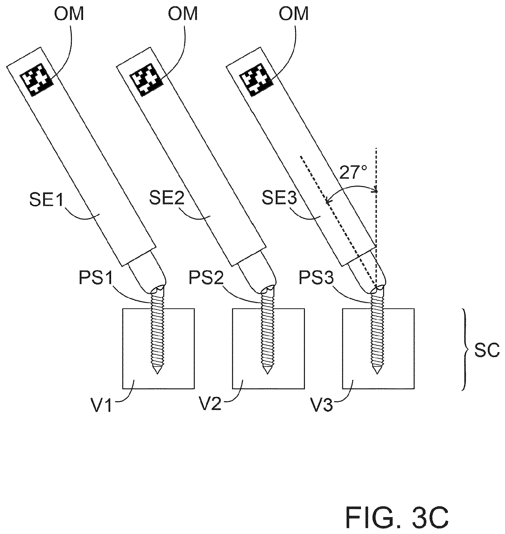

FIG. 3C shows a side view of three (3) screw extenders SE that have been moved to one predefined side, to be at the outermost angular position relative to screw head SH and bone anchor BA, for increasing an accuracy of the calculations of the positions of the vertebrae and the spinal column SC, based on a fixed geometrical relationship between screw extenders SE1 to SE3, and bone anchors BA1 to BA3 of screw heads SH1 to SH3;

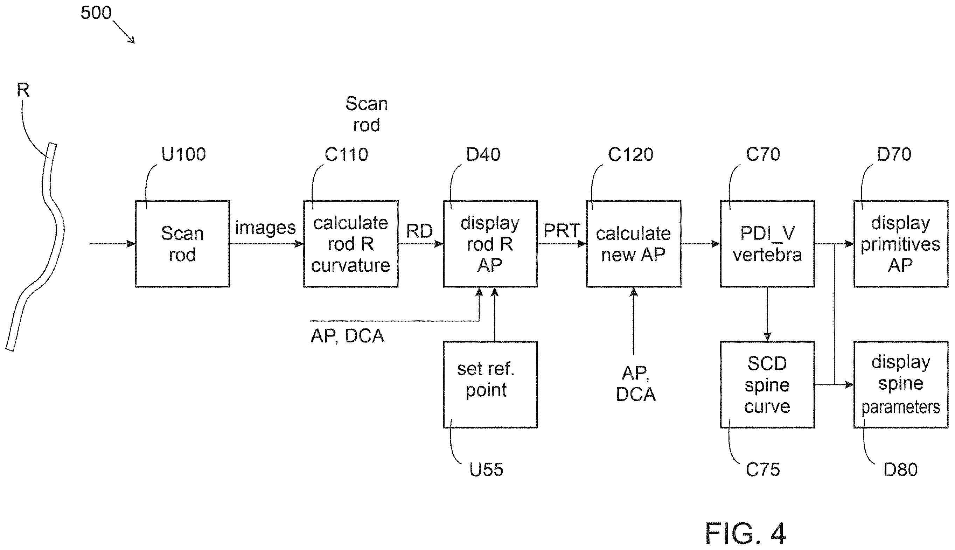

FIG. 4 shows an exemplary flowchart of a method 500 for scanning, calculating and displaying rod data RD of a real spinal stabilization rod R, and the calculation of a virtual spine correction based on the rod data RD, to visualize a virtual correction to spinal column SC, according to another aspect of the present invention;

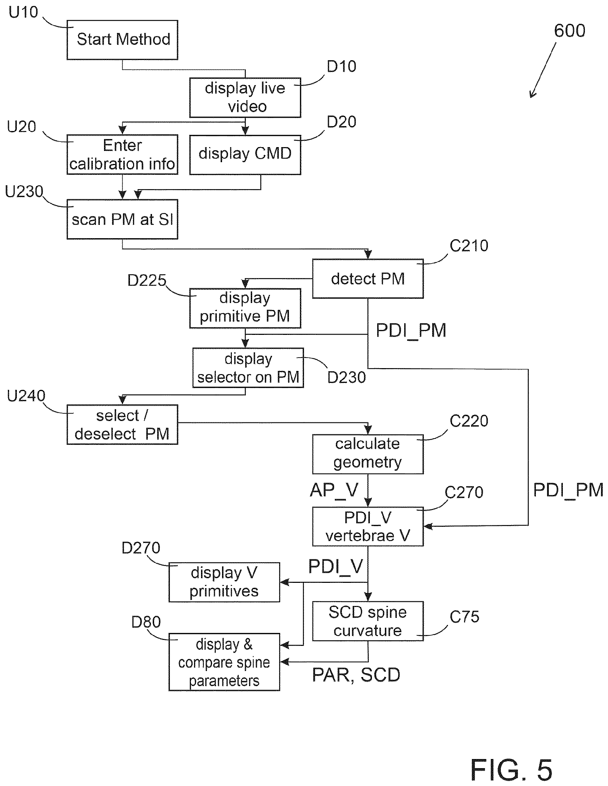

FIG. 5 shows an exemplary flowchart of a method 600 for determining different types of information that characterizes the spinal column SC before the placement of the pedicle screws PS is made, by detection of guide wires GW or pedicle markers PM visible from the surgical incision SI; and

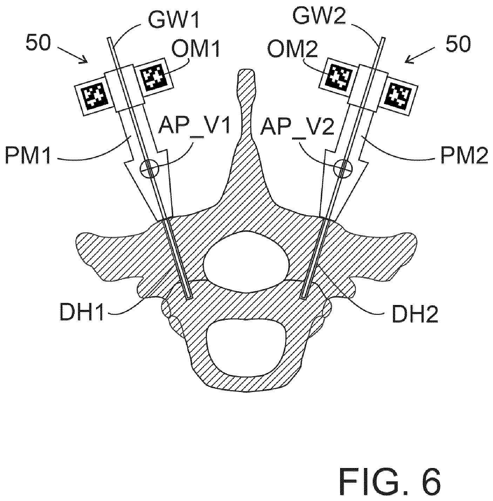

FIG. 6 shows an exemplary and simplified cross-sectional representation of a vertebrae with two drill holes DH1, DH2, two guide wires GW1, GW2 placed into drill holes DH1, DH2, respectively, and two pedicle markers PM1, PM2 attached to guide wires GW1, GW2, respectively, with optical markers OM provided with a removable or fixedly attached optical marker part 50, according to an aspect of the present invention.

Herein, identical reference numerals are used, where possible, to designate identical elements that are common to the figures. Also, the images in the drawings are simplified for illustration purposes and may not be depicted to scale.

### DETAILLED DESCRIPTION OF THE SEVERAL EMBODIMENTS

FIG. 1A shows a perspective view of a place where the orthopedic surgery is performed, showing living being or patient L on a surgical table having a surgical incision SI, and user, operator, surgeon, medical assistant O holding an exemplary data processing device 100 for performing the method for proposing different types of spinal rods for an orthopedic surgery, and FIG. 1B shows an exemplary and simplified flow chart depicting the different steps of the method for proposing different types of spinal stabilization rods for stabilizing and fusion of vertebrae of a spinal column SC of a living being or patient L, the method being performed by a computing device having an imaging capturing device and a display screen, during a spinal orthopedic surgery at a surgical incision SI by using augmented reality, according to an aspect of the present invention. As an exemplary embodiment, an orthopedic spinal surgery is shown and described, where the method is performed and used to assist the user, operator, surgeon, medical assistant O to choose an appropriate stabilization rod for attachment to two or more pedicle screws. For example, the method can propose a specific rod having a certain pre-bent shape among a plurality of rods having a different bent shapes, or can propose a specific curvature or bending curve for a rod, that can be thereafter bent by surgeon O for the surgery.

It is noted that the spinal orthopedic surgery is only exemplary in nature, and the same method using augmented reality could be used for other types of orthopedic surgery where a stabilization rod or another type of stabilization device is required for attachment to different types of pre-placed bone screws that have a detectable screw extender attached thereto, for example but not limited to fracture repair surgery requiring stabilization by a rod, or other type of fracture or reconstructive surgery using an external fixator.

Before performing the method 200, an orthopedic surgery is performed, where the surgeon O starts and performs the orthopedic surgery for example based on a customary surgical workflow. Thereby, a surgical incision SI is made to living being or patient L, and for illustration and descriptive purposes, it is assumed that at least two pedicle screws PS1, PS2, are placed to respective vertebrae of the spinal columns SC of living being L, in the variant shown three (3) pedicle screws PS1, PS2, PS3. This number is only exemplary and chosen for illustration purposes, and it is possible to perform method 200 with a different number of screw extenders SE and corresponding pedicle screws. Usually, each vertebra requires two pedicle screws on each side. Thereby, each one pedicle screws PS1, PS2, PS3 are attached to a respective screw extender SE1, SE2, SE3 with their screw head SH1, SH2, SH3. An example of at least a portion of such surgery is shown in U.S. Patent No. 10,058,355, see Figures 18-38, this reference herewith incorporated by reference in its entirety.

Once the plurality of pedicle screws PS1, PS2, PS3 are placed at their final position relative to the respective vertebra V1, V2, V3, for example by being threadably tightened with a bone anchoring element of pedicle screws PS1, PS2, PS3 to the individual vertebrae, by use of screw extenders SE1, SE2, SE3 and a screw driver, as shown in U.S. Patent No. 10,058,355, surgeon or operator O needs to choose or provide for a spinal stabilization rod R that is bent or has a shape such that it can be placed into each accommodating opening of the screw head SH1, SH2, SH3 of the plurality of pedicle screws PS1, PS2, PS3. Preferably, each screw head of the pedicle screws PS1, PS2, PS3 has a U-shaped groove for receiving the rod R, and has a threading so that the rod R can be attached to the screw head by a set screw. For determining a shape or curvature of rod R that will be placed into the screw heads SH1, SH2, SH3 of pedicle screws PS1, PS2, PS3, it is desirable to have information of the position and orientation of screw heads SH1, SH2, SH3 relative to each other, such that at least one of a shape, curvature, or length of rod R before placement and connection to screw heads SH1, SH2, SH3 can be determined.

At this stage of the surgery, surgeon O can use data processing device 100 to start method 200 for proposing different types of spinal stabilization rods for stabilizing and fusion of vertebrae of a spinal column SC of a living being or patient L. The steps of the method 200 can be performed by a dedicated application software that includes computer instructions, the computer instructions are executable on the data processor of data processing device 100 to perform the aspects of the herein described method, method 200 configured to operate and display a graphical user interface GUI with user commands, for example as a graphical overlay on the live video feed that can be shown on the display device 120 of data processing device 100. Display device 120 can be a display screen that also includes touch sensitive properties for information input, for example a touch screen. Preferably, data processing device 100 can be a portable device, for example but not limited to a smartphone, cellular phone, or tablet, or another type of handheld data processing device. Moreover, data processing device 100 can also include a graphics processor that supports image data processing and generation of live video feed and GUI, as well as other graphical elements that are displayed on the GUI.

The method 200 can be started once all pedicle screws PS1, PS2, PS3 with their respective screw extenders SE1, SE2, SE3 are placed, as exemplarily illustrated in FIGs 1A and 1B. A first step U10 is performed where the method 200 is started, for example by launching the application by surgeon or operator O. Next, method 200 proceeds to step D10 where a live video feed is generated and displayed on the GUI of the display device 120, for example the touch screen of a smartphone. This can be done by touching a button or active graphical element of the GUI for starting the live video feed that is displayed one the application, or can be started automatically upon launching of the application with step U10. Thereby, data processing device 100 stars capturing a sequence of images with the image capturing device 110, for example a smartphone built-in camera unit, and simultaneously displays such images in real-time on display screen 120, for example with a window of a graphical user interface GUI, or on a full screen of display device or screen 120. The live video feed is based on the captured images of image capturing device 110 and are displayed as a real-time video sequence, to allow for overlaying additional graphical elements, animations, and other objects for augmented reality representations. It noted that step D10 is an optional step, because it is also possible to perform the method with a transparent or semi-transparent display screen or device, for example with wearable augmented reality (AR) glasses, a head-mounted display having a transparent or semi-transparent display screen or device, or a head-up display (HUD), where the surgical incision SI can be directly viewed.

Next, optionally, method 200 proceeds to step D20 where instructions or commands CMD can be displayed or otherwise provided to surgeon or operator O, for example to request basic information for starting the method, for example by requesting calibration information, orientation information, or other type of information permitting the next scanning step U30 for manual data entry by surgeon or operator O. In addition, this step D20 can also provide for graphical elements that allow the user to enter data for method 200, specifically data related to the instructions or commands CMD. This can be done with graphical elements that are overlaid over the live video feed, for example with a text prompt, graphical prompt or one or more selection buttons. It is also possible that step D20 provides for audio information in the form of voice commands to assist surgeon or operator O to provide for instructions or commands CMD. Also, a step U20 can be performed, where the surgeon or operator O can enter data to respond to the instructions or commands CMD, as exemplarily shown with the screen shot of FIG. 2A. These steps can be performed at least partially simultaneously while surgeon or operator O continuously films the surgical incision SI to generate the live video feed of step D10, permitting the display of commands and buttons CMD. For example, in the variant shown, step D20 displays text box for surgeon or operator O requesting from information on an orientation of living being L relative to the live video feed view, with a step D20 where a text box is displayed in the GUI, providing surgeon or operator O with additional information related to the information request. Also, step D20 can display and overlay two symbolized heads with a graphical icon on the left side and on the ride side of the GUI, so that surgeon or operator O can select one of these two graphical icons to indicate what side the head of the living being L is located, relative to the position of the data processing device 100. Once one the graphical icons that represent the heads is selected, the head can be highlighted, and the requested information can be confirmed with a confirmation button overlaid over the live video feed of the GUI, as shown in FIG. 2A.

Generally, in the context of the present description, the steps where surgeon or operator O enters data to method 200 or other methods described herein, for example but not limited to step U20, the requested data can be entered by the use of a voice or speech recognition software that is operated on data processing device 100, using a microphone 130 of data processing device 100, instead of entering data manually by touching graphical elements such as buttons on a GUI with a touch screen operation. This would allow surgeon or operator O to provide for data or information to respond to the instructions or commands CMD by voice commands, and such implementation of voice and speed recognition would allow surgeon or operator O to at least partially avoid touching the display screen 120 during method 200. The entering of the data can thereafter be confirmed by audio, for example with a voice prompt, or with different graphical elements that are displayed, using one or more speakers that are part of or operatively connected to data processing device 100.

However, it is also possible that this step U20 is automated by computer-based process, for example by an image data processing algorithm with data processor and memory of data processing device 100 is performed to detect an orientation of living being L relative to the captured sequences of images that provide for the image data for the live video feed. As an example, this can be for example by the use of an trained neural network that can detect orientation of living being L based on training data, or by the use of optical markers that are attached to living being L or the medical or surgical bed or table, as further explained below with respect to optical markers OM that are attached to screw extenders SE, or by detecting an orientation of the medical or surgical bed or table that can be detected by a pattern matching algorithm, providing for information related to the orientation of living being L.

As explained above with step U20 and D20, simultaneously with the displaying of the live video feed of step D 10 with a GUI, different user commands and information can be displayed on the GUI at different moments during the performance of method 200, for providing user direction, and for receiving user information and instructions. For example, different text prompts or text boxes with text information can be displayed as an overlay over the live video feed, giving surgeon or operator O information on the type of processing that is performed or status information of the method 200, as feedback to surgeon or operator O, or requesting user input via icons or buttons. It is also possible that the different information and instructions is requested by audio, for example voice prompts. Also, it is possible that a graphical element or icon is displayed that allows opening or pulling down a menu for configuring method 200, for example by locking image quality and image capturing parameters and features, for example but not limited to zooming or image clipping, automatic image corrections settings, auto color and white balance adjustments, wide angle settings. Also it is possible to provide for a graphical icon that can be touched or otherwise selected by surgeon or operator O, to revert back to the previous step of the method 200.

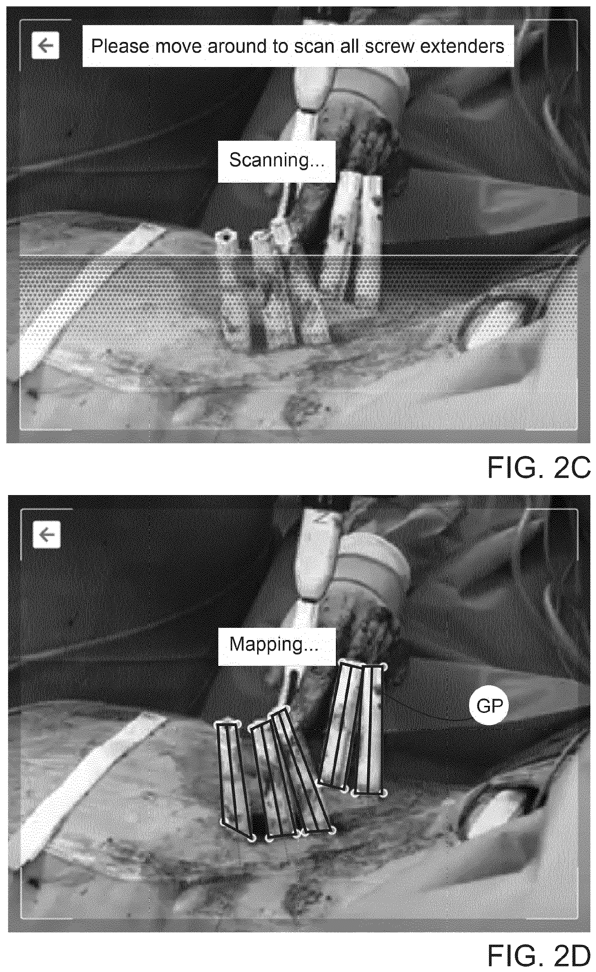

Next, in a step U30 of scanning the screw extenders SE, surgeon or operator O is informed or encouraged, for example by a text prompt, to film and capture image sequences of the screw extenders SE that point out of the surgical incision SI while the live video feedback is displayed on GUI with a continuous performance of step D10. An exemplary screenshot of this step is shown in FIG. 2B, where a text box requests user or operator O to move data processing device 100 to scan all of the screw extenders SE. Next, with step D10, surgeon or operator O directs the viewing angle and viewing field of image capturing device 110 towards living being L and surgical incision SI, such that all screw extenders SE, for example the three exemplary screw extenders SE1, SE2, SE3 can be seen in the live video feed of the GUI, to capture the image sequences at and around surgical incision SI. This is exemplarily shown in FIGs. 2C with a screen shot, where a text box can be displayed that indicates that the scanning of the screw extenders is in process. With FIG. 2C it can also be seen that the field of view of image capturing device 110 that is displayed with GUI shows five (5) screw extenders SE, three (3) in the front that are attached to the right side of the pedicle bone on the back of the spinal column, into the vertebral body, and two (2) on the left side.

Simultaneously or after the step of scanning U30 has been started, while surgeon or operator O is still filming surgical incision SI and screw extenders SE, an image data processing step C10 is performed, a step of detecting the different screw extenders SE. This can be done by different types of image processing algorithms that are performed on the captured images. For example, this can be done by a step-by-step method where first one screw extender SE is searched for, detected, and its data saved, for example by a three-dimensional coordinate data model. Thereafter, the next screw extender SE is searched for, detected, and its data saved, and these substeps are repeated until all screw extenders SE in the field of view of image capturing device 110 are detected and saved. Preferably, the detection step C10 is performed while the data processing device 100 is moving, which means that the angle of view and viewing window of screw extenders SE and surgical incision SI is variable and changing.

As an example, the detecting step C10 can performed by using a rigid body model-based three-dimensional (3D) pose, positioning estimation algorithm and tracking algorithm, to detect and track the shape of the screw extender SE, and thereafter extract a pose of the screw extender SE, to provide for a data set of pose data information PDI. Because all of the shapes of the screw extenders SE are known, being all the same shape and dimensions, a three-dimensional model can be used for this detecting step, for example a computer aided design (CAD) data model. After detection of one of the plurality of screw extenders SE, the step generates pose data information PDI that can be stored and updated, the pose data information PDI can include a coordinate reference position, angular and rotational orientation of the screw extender SE, for example different vectors, referenced to a coordinate system, for example a real-world coordinate system such as an Euclidian space. It is also possible that pose data information PDI only includes a coordinate position of the different screw extenders SE to simplify the calculations. Pose data information PDI can be calculated in different forms and coordinate spaces, but in the preferred embodiment, the coordinate data is three-dimensional data that is references to the Euclidian coordinate space. Once all of the pose data information PDI for all screw extenders SE is generated, a data set or table can be generated with a data set of all the gathered pose data information PDI. Whilst the body shape of screw extenders SE is known, screw extenders SE are only partially visible in the image sequences, because the front end of screw extenders SE is interconnected with heads of pedicle screws PS inside the surgical incision, as can be seen in the exemplary screen shot of FIG. 2C. For this reason a robust algorithm for computer vision tracking needs to be performed where also partial shapes of the body can be detected, and pose data information PDI be generated. As every surgical incision is a priori not known and can vary largely between different incision locations, living beings L, arrangement of surgical tools, preferably the vision tracking algorithm is used that does not require a pre-knowledge of the scene-to-be-tracked.

An example for a model-based tracking algorithm that can be used is described with U.S. Patent Publication No. 2019/0355150, this reference herewith incorporated by reference in its entirety, where trained neural network is used for object detection. With a trained neural network, for example but not limited to a CNN and the deep learning based on images of screw extenders with known pose data information PDI, training data can be established that allows to directly link partial views of a screw extender SE to its pose data information PDI. As another example, the robust and model-based augmented reality tracking algorithms from the company visionLib^{™} from Visometry GmbH can be used.

As another example, the detecting step C10 can be performed by using a contour detection algorithm, that allows to first detect a contour of each one of the screw extenders SE, and thereafter map each detected contour to a two-dimensional (2D) projection of a three-dimensional (3D) model of the screw extenders, to thereafter determine a data set of the pose data information PDI.

With the step of detecting C10, the detected screw extenders SE can also be tracked and updated during the capturing and displaying image on the live video feed. This may be necessary as the surgeon or operator O will move a position and direction of the filming of the screw extenders SE with the step U30 of scanning, and thereby more information will be gathered to further refine the data set of the pose data information PDI. However, it is also possible that the screw extenders SE themselves can move slightly relative to each other, which can change their coordinate and orientation data. In this respect, the data set of the pose data information PDI may change as a function of time, and the data structure or table that contains the data set of the pose data information PDI can be regularly updated during the step of detecting C10.

In a variant, the step C10 does not require the placement of any screw extenders SE to the pedicle screws PS, and is performed simply with the pedicle screws PS that are attached to the vertebrae V. For example, a screw head SH of each pedicle screw PS could have an optical marker OM printed, etched, engraved, patterned or otherwise provided thereon, for a more robust detection of screw head SH of each pedicle screw PS by tracking algorithms. For example, optical marker OM could be such that it has some redundant information, so that it can still be detected even if marker OM is covered with flesh, muscles, fat, or other body parts of the surgical incision SI. See for example Köhler et al., "Robust Detection and Identification of Partially Occluded Circular Markers," In International Conference on Computer Vision Theory and Applications (VISAPP), Vol. 1, pp. 387-392, year 2010. See also, ARTag fiducial optical marker. In a variant, before the step C10 of scanning the screw heads SH instead of the screw extenders SE, each screw head SH is equipped with a removable optical marker part 50 having an optical marker OM arranged thereon, as shown in FIG. 1E, but this time not placed onto the screw extender SE, but directly placed onto each screw head SH of the pedicle screws PS. The interconnection between optical marker part 50 and screw head SH could be done by configuring an end portion of optical marker part 50 with a complementary or corresponding interconnection element to the one of the screw head SH of pedicle screw PS, for example a press-fit engagement as discussed in U.S. Pat. No. 10,058,355, or a snap-lock, or other type of geometrically-defined lock between optical marker part 50 and the screw head SH, so that the optical marker part 50 can be connected to screw head SH at a precisely defined position, and yet is still easily removable, as only being used for the detection. Thereby, the geometric relationship between the optical marker OM, optical marker part 50, and the screw head SH of pedicle screw PS, in the interconnected state or position, is defined and not variable. This would reduce or even entirely obviate the problems of detection reliability for the pedicle screws PS. However, it is also possible that at least parts of method rely on the detection of pedicle screws PS with image processing algorithms without any additional optical detection aids.

As another variant of step C10, each pedicle screw PS can be equipped with one or more radio-frequency identification tag (RFID) that allows for a detection of a three-dimension position in space, based on different detection techniques and RFID detection antenna use, preferably passive RFID tags. For example, this can be done by using RFID tag arrays that are attached to pedicle screw PS, for example screw head SH, with different RFID tags having a different orientation to each other, for example a plurality of RFID tags that are oriented with the different axes of a three-dimensional coordinate space, and using an RFID detection antenna that can move relative to the RFID tags of the pedicle screws PS, for improved position accuracy. See for example Zhang et al., "3-Dimensional Localization via RFID Tag Array," In 2017 IEEE 14th International Conference on mobile ad hoc and sensor systems (MASS), pp. 353-361. IEEE, year 2017. It can also be envisaged that a plurality of reference RFID tags are used, not attached to the pedicle screw PS to provide for different known reference positions, for example arranged in a matrix, and one or more RFID tags are attached to the pedicle screws PS, for example the screw head SH, for example see Liu et al., "A Three-Dimensional Localization Algorithm for Passive Radio-Fequency Identification Device Tag," International Journal of Distributed Sensor Networks, Vol. 13, No. 10, year 2017, ref. 1550147717736176.

As another variant, ultra-wideband RFID tags can be used, and detected by different types of detection algorithms, for example by backscatter modulation or UHF and UWB modulations, using a plurality or reader antennas, see for example Dardari et al., "Ultrawide Bandwidth RFID: The Next Generation?," Proceedings of the IEEE Vol. 98, No. 9, year 2010, pp. 1570-1582. In such case, different elements such as reference RFID tags, one or more reader antennas, and a data processing device for performing the data processing algorithms on the read signals from RFID tags could be part of the system shown in FIG. 1A, and interconnected to data processing device 100 to deliver coordinate data of screw heads SH and attachment positions AP, to deliver the reference frame information or other data that allows to find the of screw heads SH and attachment positions AP in a specific reference frame, for further processing by method 200. However, it is also possible that raw data of the reader antennas is provided to data processing device 100 over a network to determine the coordinate positions of screw heads SH and attachment points AP at device 100.

As yet another variant, screw heads SH can be detected by thermal imaging, based on the premise that the screw heads SH of pedicle screws will be colder that the environment in the surgical incision SI, specifically due to different thermal radiation that emits from metallic screw heads and the surrounding tissue of surgical incision SI. For example, infrared thermography can be used for measuring infrared energy produced from exposed tissue and bone of a surgical incision SI and of implants such as pedicle screws PS and their screw heads SH, and this infrared energy can be converted into a radiometric thermal image indicating a distribution of surface temperature. Such images can be subject to image data processing algorithms for detecting the screw heads SH, or even for detecting screw extenders SE. An exemplary thermal imaging camera that could be used for this purpose is an infrared (IR) thermographic camera FLIR T335 from the company FLIR Systems Inc. This would also require the use of a reference marker or reference frame that could be seen by the thermal imaging camera (not shown) and also by image capturing device 110 operating in the visible light range, to provide for a reference position for the coordinates, for example a ruler, markers, etc. Based on image processing algorithms, screw heads SH or screw extenders SE can thereby be localized and detected from thermal images, for example by a model-based pattern matching algorithm, or by other type of artificial-intelligence based detection algorithms. For this purpose, system as shown in FIG. 1A would further include a thermal imaging camera that can provide for thermal imaging data to a data processing device over a network, for example to data processor 100.

This information on the position of either screw extenders SE or position of screw heads SH can be used by steps D25, D30, and U40, to provide for the graphical primitives GP that can be overlaid over the live video feed to highlight the different screw extenders SE, or in a variant graphical primitives GP to highlight the different detected screw heads SH of the pedicle screws PS, if no screw extenders SE are placed thereon, for the selection and deselection of different pedicle screws PS that need to be considered for the geometry and rod template calculation of steps C20, C30.

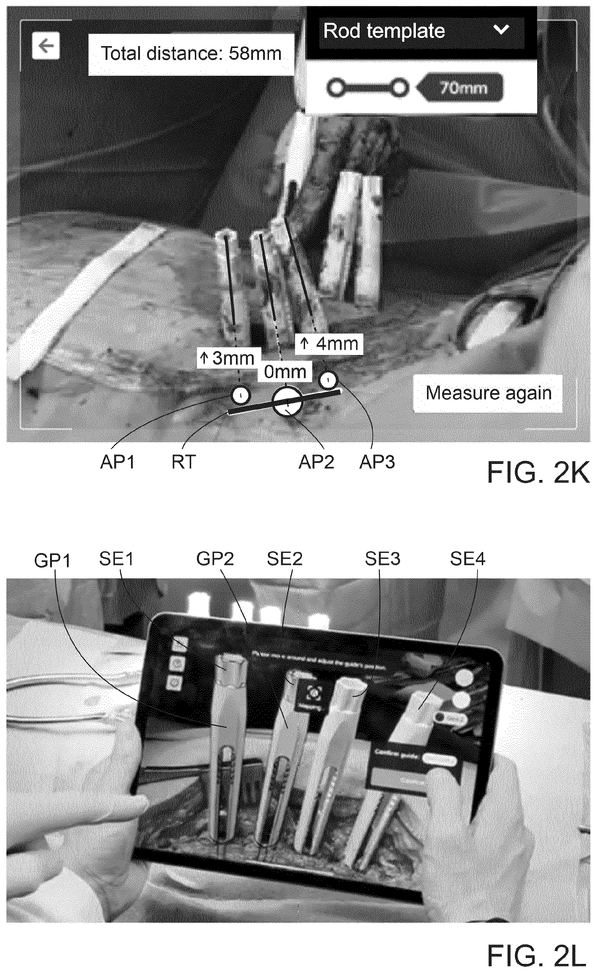

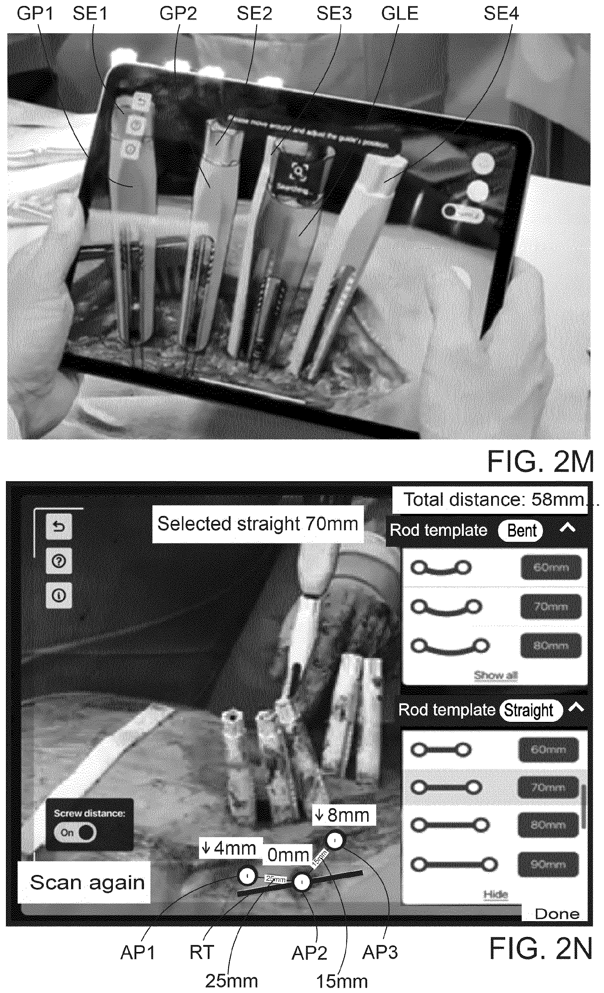

Moreover, in a variant, the step U30 of scanning, the step of detecting C10, and the step of displaying a primitive D25 can be iteratively performed and thereby repeated, for example for each detected screw extender SE. This variant is illustrated with the representations of FIG. 2L and 2M, where an exemplary number of four (4) screw extenders SE1 to SE4 are tracked and detected. For example, the step of scanning U30, the searching and scanning of the screw extenders SE can be further aided by using a graphical locator element GLE that is displayed and overlayed over the live video feed, see for example FIG. 2M. For example, a graphical locator element GLE that be displayed that represents a graphical representation of a screw extender, for example a graphical representation of a rendering or projection of a screw extender SE on the screen, an outline of a screw extender SE, for example a semi-transparent graphical representation of a screw extender SE can be shown on the screen, or other type of graphical locator element GLE that can be used as a locator to scan and detect screw extenders SE, for example a crosshair, a reticle, cursor, arrow, indicator. For example, this graphical locator element GLE can be presented on screen at a fixed location relative to screen, for example substantially in a center of the displayed field of view. This allows surgeon or operator O to move device 100 with step U30 to thereby also move the filmed or captured scene with surgical incision SI relative to the graphical locator element that is filmed by image capturing device 110 of data processing device 100.

In the variant shown in FIG. 2M, graphical locator element GLE is fixedly represented in an upright position in the center of the screen of the GUI, rendered as a semi-transparent element as an outline of a screw extender SE over the live video feed, having a position and orientation that allows operator O to move device 100 such that graphical locator element GLE can be matched with one of the screw extenders SE1 to SE2 that are protruding from the surgical incision SI. In the variant shown, the longitudinal grooves of screw extender are also represented in graphical locator element GLE, thereby serving as an orientation aid to operator O with respect to an angle of orientation to hold and move device 100 to detect screw extenders SE1 to SE4.

Upon partial or full visual contact or touching of the graphical locator element GLE with one of screw extenders SE1 to SE4 captured by the video feed, the thereby contacted screw extender SE can be detected with step C10, and thereafter highlighted, for example by displaying a primitive for the detected screw extender SE with step D25. For example, the detection step C10 can be split into a course detection step C12 that is performed simultaneously with the step of scanning U30, where the touching or contact of the graphical locator element GLE with a screw extender SE can be detected. This course detection step C12 can be based on a pattern matching algorithm or other types of detection algorithms that allows to detect surfaces or areas in the current image where screw extenders SE are located, and thereafter, when coordinates or an area of graphical locator element GLE is in contact, in proximity, or touches an area of the image that represents the screw extender SE, a fine detection step C14 can be performed, where the exact location and coordinates of screw extender SE are detected, for example the pose data information PDI. Upon full detection of screw extender SE with step C14, the augmented reality graphical primitives GP1 to GP4 can be displayed on the detected screw extender SE, and the surgeon or operator can be prompted to accept the detection of the screw extender SE, to thereby also accept the detected pose data information PDI, for example with a prompt, text box, confirmation button, as shown in FIG. 2L. With the exemplary view of FIG. 2M, two of the screw extenders SE1 and SE2 arranged on the left side have already been detected and are displayed overlayed with a graphical primitive GP1 and GP2, and the graphical locator element GLE is displayed in the center of the image, the graphical locator element GLE showing a rendering of a screw extender SE that is semi-transparent.

In a variant, for the detection, as visualized in FIGs. 2L and 2M, upon an approximate match of the graphical locator element GLE having a specific pose information on position and orientation with the coordinates fixed relative to the screen 120 or device 100, with pose data information of a tracked one of the screw extenders SE1 to SE4 of surgical incision SI, the matching screw extender can be highlighted for selection. In this respect, upon an exact or approximate match of pose data information PDI of one of the screw extender SE1 to SE4, the detected one can be overlayed with a graphical primitive GP, and a confirmation prompt can be presented to operator O, as shown in FIG. 2L. For operator O to cause the matching of the PDI between one of screw extenders SE1 to SE4 and the PDI of graphical locator element GLE, he or she has to move device 100, for example by turning, tilting, moving, until the displayed graphical locator element GLE is approximatively matching with one of screw extenders SE, in FIG. 2M being SE3. In this step, for example by a pattern matching algorithm, coarse PDI information for different screw extender candidates for selection can be repeatedly calculated until a match is found with PDI of graphical locator element GLE.

Next, the steps U30 of scanning, detecting C10, and displaying a primitive D25 can be repeated for the next screw extender SE, and successively, one after the other of the screw extenders SE1 ro SE4 are detected and highlighted by the primitives, until all the desired screw extenders SE are detected, as illustrated in FIG. 2D. This variant is a step-wise scanning that allows to provide for a direct visual and intuitive feedback to surgeon or operator for detection of each screw extender SE. The detection moment or instant of step S14 per screw extender where the PDT of the SE are captured and tracked can also be further emphasized by a signal, for example an auditory or vibratory signal, or both.

In a variant, screw extenders SE are not detected by a computer vision algorithms that detect a shape, contour, or a pattern as described above, but each screw extender SE is equipped with an optical marker OM that can be detected and tracked with the step of detecting, and optical markers OM can also serve as fiducial markers for the viewed scenery. An example of such viewed scenery with the surgical incision SI is shown in FIG. 1D, showing two rows of screw extenders SE1-SE6 that are attached to pedicle screws PS (not shown). In the example shown, each screw extender SE can be equipped with two optical markers OM, at different locations, to provide for a more robust detection of the screw extenders, such that a first marker OM is located at a distal end of the body of screw extender SE, and a second marker is located in a middle section of the body of screw extender SE. In case one of the two markers is covered from the camera view, as shown for example with screw extenders SE4, SE6, there is a detection and tracking redundancy provided with the other visible optical marker OM.

Each screw extender SE can be equipped with a plurality of optical markers OM for redundancy purposes, as some markers OM may be placed that they are hidden inside the surgical incision, covered up by other screw extenders SE, or are otherwise outside of the field of view of image capturing device 110. It is also possible that surgeon or operator O visually inspects whether the screw extenders SE are being detected, and can move his filming and viewing position of camera or imaging device 110, so that at least one marker OM is detected and tracked. In the example of FIG. 1D, for the detection and tracking of screw extender with step C10, a different camera view may be required to detect at least one optical marker OM5.

The optical marker OM can be made of a graphical pattern or design that has a fixed geometric relationship to screw extender SE, for example by being placed at a specific location with a specific orientation. As an example for the pattern that can be used for an optical marker, it can be a checkerboard pattern, matrix code or QR code, or similar design, for example designs that are used in tracking for robotics. Different tracking markers, such as but not limited to the ARToolKit, ARTag, AprilTag, and ArUco fiducial tracking markers are an example of optical markers OM that can be used to label the screw extenders SE, and they are useful for both identification and pose estimation purposes. For example, each screw extender SE can be fixedly equipped with one or more optical markers OM, for example by printing, sticking, etching, embossing, grating, or deposition of a layer having such optical marker OM. These optical markers can also be made invisible to a human eye, for example by using UV visible ink, NIR visible ink. For example, optical markers OM can be made as a removable or fixedly attached layer or sticker.

As an alternative, as illustrated in FIG. 1E, it is possible that the optical markers are a part 50 that is separate from each screw extender SE, and can be placed at a predefined geometric relationship to screw extender SE, for example by placing optical marker part 50 onto a handle attachment part 60 at the distal end of screw extender SE. Also, optical marker part 50 includes an optical marker OM, and can be easily removable form screw extender 50, once the method 100 has been performed and concluded, for example by a simple manual operation. For this purpose, optical marker part 50 can have an attachment device 55 that is complementary to the handle attachment part 60 of screw extender 60. Preferably, attachment device 55 and handle attachment part 60 are formed such that attachment device 55 can only take one position relative to screw extender SE, so that correct pose data information PDI of the screw extender SE can be calculated, taking a correct orientation of screw extender SE into account. Optical marker parts 50 can be placed onto all screw extenders SE before the step U30 of scanning the screw extenders SE is performed, for detection and tracking by step C10. As explained above, it is also possible that an optical marker OM is used having redundant information so that a detection of partially occluded markers OM is possible, for example ARTag, TriCode, ARToolkit+, Köhler circular markers.

It is also possible that for each screw extender SE, the pattern or other graphical element that is included in optical marker OM is different, and includes information that can be read and identified in an optional step of identifying C15. This information can be used for verification purposes to see if the right screw extender SE for the correct surgical operation is being used. For example, with a database, identification of each screw extender SE can be read, and different aspects of the screw extender SE could be checked, for example whether the screw extender SE has exceeded its lifetime or lifecycle, whether a correct type of screw extender SE is being used for the particular surgery. In addition, the identification information that is included in each optical marker OM can be used to identify the respective screw extender SE over several images that are captured from the surgical scenery, thereby permitting a fast calculation of a correspondence of detected screw extenders SE within a sequence of captured images. This allows to provide for a more robust and fast identification of the individual screw extenders SE over several captured images.

Once at least one of the screw extenders SE is detected, a graphical primitive GP can be generated and overlayed over the actually displayed screw extender in the live video feed of the GUI, with a step D25 of calculating and displaying screw extender primitives, as shown exemplarily in the screen shot of FIG. 2D. In this figure, a graphical primitive showing an outline of the visible part of the screw extender SE is shown, with a graphical element highlighting or indicating the corner points. The graphical primitive GP can be displayed such that covers or otherwise graphically indicates a location of a respective screw extender SE during the live video feed, to provide for the augmented reality of a computer-generated graphical element for the screw extenders SE, and the real world. This can be done by calculating data that represents a camera position and orientation for the currently captured and displayed image, relative to the screw extenders SE, and by calculating and displaying a projection of the screw extenders SE as a graphical element for the graphical primitive GP, based on the data set of the pose data information PDI. However, step D25 of calculating and displaying screw extender graphical primitives GP can also be done entirely separately from the pose data information PDI that has been gathered by the step of detecting C10, and can be based on a contour detection algorithm that detects an outline of the screw extenders SE, and thereafter graphically displays an element, for example but not limited to lines, shadings, dots, points, boxes.

Generally, step D25 allows to provide for computer-generated information on the screw This step preferably includes two substeps, with a first substep that detects fixed interest points, fiducial markers, or optical flow on the captured images of the live video feed. detect interest points, fiducial markers or optical flow in the camera images. Thereby, the first substep can create an orientation data model of the current camera view. This step can use feature detection methods like corner detection, blob detection, edge detection or thresholding, and other types image processing methods. The second substep restores the real-world coordinate system of the currently filmed environment, being the surgical incision SI and the screw extenders SE. As at least a part of the viewed scenery with surgical incision SI and body is unknown, simultaneous localization and mapping (SLAM) can map relative positions of pose data information SPI to screen location coordinate data SLCD can be calculated, for displaying the graphical primitives GP at the correct location on the live video feed. In this respect, a graphical representation of a screw extender SE can be a projection view of a geometric model of a screw extender, projected to the screen location coordinate data SLCD. In addition or alternatively, structure of the viewed scene can be derived from motion methods like bundle adjustment are used, and the mathematical methods used can include the use if projective (epipolar) geometry, geometric algebra, rotation representation with exponential map, Kalman and particle filters, nonlinear optimization, and robust statistics. With this step D25, a graphical representation of a real word object, in the present case the graphical primitive GP of screw extender SE, is correlated with a real-word view or scene of the screw extenders SE. It is also possible that the viewed scenery is further analyzed for the mapping based on a three-dimensional information, for example based on data from a distance measurement sensor includes a direct time of flight (dToF), LiDAR sensor, or a structure light sensor, stereo imaging with two image sensors. The graphical primitive GP can be considered a virtual reconstructed and projected model of the screw extender SE. An example of the implementation of such step D25 can be found with U.S. Patent Nos. 10,824,310, and 9,824,495 these references herewith incorporated by reference in its entirety.

With this step D25, a visual feedback can be given to surgeon or operator O to see if all the screw extenders SE have been detected, and provide for one aspect of the augmented reality concept as a live video feed of surgical incision SI is further augmented with dynamically moving graphical elements for highlighting the screw extenders SE. In a variant, first all screw extenders SE are detected and the pose data information PDI extracted and stored into a table or data structure, and thereafter a graphical primitive GP is overlayed over the screw extenders. This step can be performed simultaneously with the scanning step U30 and the detecting step C10. For example, the graphical primitive GP can be a non-transparent, transparent, or translucid shading that covers the respective screw extenders SE that have been detected.

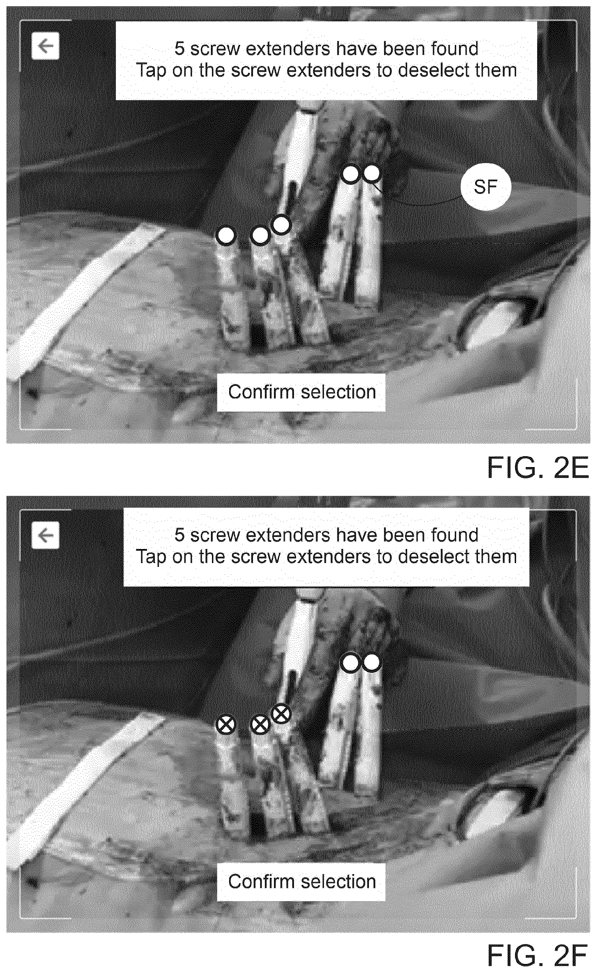

Next, method 200 can perform a step D30, where a selector element SF is generated and displayed over the live video feed for each detected screw extender SE, and a step U40 is performed of selecting or deselected screw extenders SE by selector element SF, allowing the surgeon or operator O to manually select individual ones of the screw extenders, preferably by a touch screen operation. Step D30 thereby provides for another aspect of the augmented reality concept, allowing for an easy interaction with surgeon or operator O to select or unselect screw extenders SE that will be taken into account, and with step U40 the use of graphical element SF on a GUI for the selection/deselection of screw extenders that are placed on the live video feed, for example by the touching with a finger of surgeon or operator O of selector elements SF for toggling the selection/deselection. Also, the graphical representations of selector elements SF can be moved to be located or point to the respective screw extender SE, even when the viewing angle change. Step D30 of displaying selector elements SF can display a graphical element on the GUI, for example but not limited to a field, box, arrow, icon, label or other type of graphically selectable labeling or element for each one of the screw extenders SE, dynamically overlayed over the live video feed, as exemplarily shown in the screen shot of FIG. 2E, for example by linking the display coordinates of selector elements SF with display coordinates of a respective graphical primitive GP that has be generated by step D25, or by calculating a projection from pose data information PDI of the screw extenders SE. This graphical overlay of selector elements SF over the live video feed allows surgeon or operator O to select active ones of the screw extenders SE, for proposing a rod template RT, or for selecting a rod template among a plurality of rod templates, as further explained below. For example, surgeon or operator O may want to select the front row with three (3) screw extenders SE for the rod determination.

The selection made by surgeon or operator O with step U40 can be confirmed by a graphically displayed confirmation button that can be accessed by a touch screen operation, and can also be guided by text boxes having information on how many screw extenders SE have been detected, having information on how many screw extenders have been selected, as also exemplarily shown in FIG. 2F, where the front row of three (3) screw extenders has been selected. The selection or deselection of a screw extender with SF can also be highlighted or de-highlighted by a graphical element, so that a visual feedback is provided to surgeon or operator O with respect to the status of the selected SE.

Upon confirming the selection of screw extenders with step U40, method 200 proceeds to step C20 of calculating a geometry of the rod attachment locations of the pedicle screws PS is performed. For example, in this step, the geometry can include coordinate data of all attachment center points AP for a spinal stabilization or fixation rod R, and these can be calculated based on data of the detected and selected screw extenders SE from steps C10 and U40. In the variant described herein, this step determines an attachment center point AP for each pedicle screw PS of imaginarily-placed or fictional rod R for each selected screw extender SE, each pedicle screw PS being attached to a corresponding screw extender SE, taking into account that spinal stabilization or fixation rod R is reduced and placed into the screw head SH at its final position for spinal stabilization, as illustrated in an exemplary embodiment in FIG. 1C. This rod is considered imaginary or fictional as it has not yet been placed to the pedicle screws PS of surgical incision. In the variant shown, the attachment center point AP for rod R is defined as being a crossing point between a center axis CA of screw head SH of pedicle screw PS and the screw extender SE assembly, and a rotational center axis of rod R, when rod R is fully placed into the U-shaped groove UG of screw head SH. However, pending on the screw head type and other considerations, attachment center points AP can be defined differently. By determining all attachment center points AP in a geometric space with step U40, for example the three-dimensional Euclidian space, it is possible thereafter to make a proposal of a rod shape or template RT for placement and attachment to the screw heads SH of the pedicle screws PS.

Step C20 can perform the geometric calculations based on the data set of the pose data information PDI that has been calculated by step C10 of detecting the screw extenders SE. It can be assumed that screw head SH of pedicle screw PS has a fixed position relative to a corresponding screw extender SE to which pedicle screw PS is removably attached to, as the screw head SH is usually fully inserted or has a fixed attachment position relative to screw extender SE. Thereby, with the coordinate and orientation data from pose data information PDI of each selected screw head, it is possible to calculate the three-dimensional coordinate position for attachment center points AP. It is noted that pedicle screws PS cannot or are only partially visible from the outside of living being L due to their placement into the surgical incision SI, but based on the detection of the placed screw extenders SE, it is still possible to calculate attachment center points AP. For example, this could be done by using cartesian coordinates, line equations, distance calculations, and surface equations, to determine the coordinates of the different attachment center points AP. For example, this can be done by first determining a line equation for center axis CA of the corresponding screw extender SE, by using the pose information of the screw extender SE, and by calculating attachment center points AP at a fixed distance from a fixed location that is the same of all screw extenders SE, calculate a location of the attachment center points AP.

Additional information that is relevant for determining a proposed rod template RT can be calculated, other than the attachment center points AP. For example, with the variant of FIGs. 1A and 2F where a rod template RT for three (3) screw extenders SE1, SE2, SE3 is being determined, not only the three coordinate points for the AP could be used for the geometry, but the geometry of the rod attachment locations can further include an orientation of screw head SH in the coordinate space, for example expressed by a direction or axis of the center axis DCA of the fictional rod R that is placed into U-shaped groove. Based on the pose data information PDI of screw extender SE, for each one of the attachment center points AP, data representing a direction of the center axis DCA of the fictional rod R can be calculated. This direction corresponds to a direction of groove extension of the U-shaped groove of screw head SH, as generally screw head SH is rigidly attached to the corresponding screw extender SE, such that a center axis of screw head SH coincides with a center axis CA of screw extender SE, while the bone anchor part of pedicle screw PS may have different orientation due to its polyaxiality.

In a variant the calculation of the geometry can also be part of another step, and could have been previously calculated, for example it could be part of the detection of the screw extenders SE with step C10 after data on the positioning or pose of the screw extenders are available, and the herein proposed order of the steps of method 200 is exemplary only.

Next, in a step C30, a data set representing a geometry for one or more rod templates RT can be calculated, herein referred to as rod template data RTD, based on the geometry of the rod attachment locations that has been determined by step C20, the geometry of the rod attachment locations including for example the determined attachment center points AP and/or a direction of the center axis DCA. For example, taken the coordinate data of attachment center points AP and a direction of the center axis DCA, an appropriate geometry for a rod template RT can be determined, for example one that could be considered the best fitting for the current position of the attachment center points AP, for example by using a curve fitting algorithm, for example a curve fitting that provides for geometric fit to attachment center points AP, or also taking into account a direction of the center axis DCA, from step C20, or a fitting algorithm that takes the bending limitations of a real physical spinal stabilization rod R and its physical limitations into account, for example but not limited to a minimally possible or allowed bending radius, maximal curvature, maximal lateral dimensions of bent rod R. It is also possible that the rod template data RTD is determined in the three-dimensional coordinate space as a series of interpolated and discrete three-dimensional points that are located between adjacent attachment center points AP. In this step C30, it is also possible to calculate a total length of rod templates RT, and the calculated length can be stored to the rod template data RTD.

According to another aspect, in a step D42, it is also possible that a window or other graphical element is displayed on the graphical user interface of the display, to show a selected rod template RT at a one-to-one scale of the real physical embodiment of the rod. This could be done by double-clicking or otherwise selecting a rod template RT, or the calculated one for best mechanical fit, for example a rod template RT from the list by a graphical button, context menu item, or other selection operation with the graphical user interface. This would allow surgeon or operator to directly compare real, physical rod R with the rod template RT that is displayed to scale, just by holding the real, physical rod R over the display screen, and the operator or user could switch between different precalculated or determined rod templates RT to graphically verify their fitting and suitability. Also, in a variant, the rod shape of the rod template RT could be bent, stretched or otherwise deformed or changed in shape by a touchscreen operation, for example by moving parts of the graphical element that shows the rod template RT sideways with a finger operation on the touchscreen. The modified virtual rod template RT can again be displayed with respect to a chosen reference or zero point, for example one of the attachment points AP1, AP2, AP3, and the offset distances from the respective attachment points can be recalculated. This step of displaying D42 and the recalculating of the parameters related to the rod template RT can be repeated, until the operator or surgeon O is satisfied with the rod template RT for use.

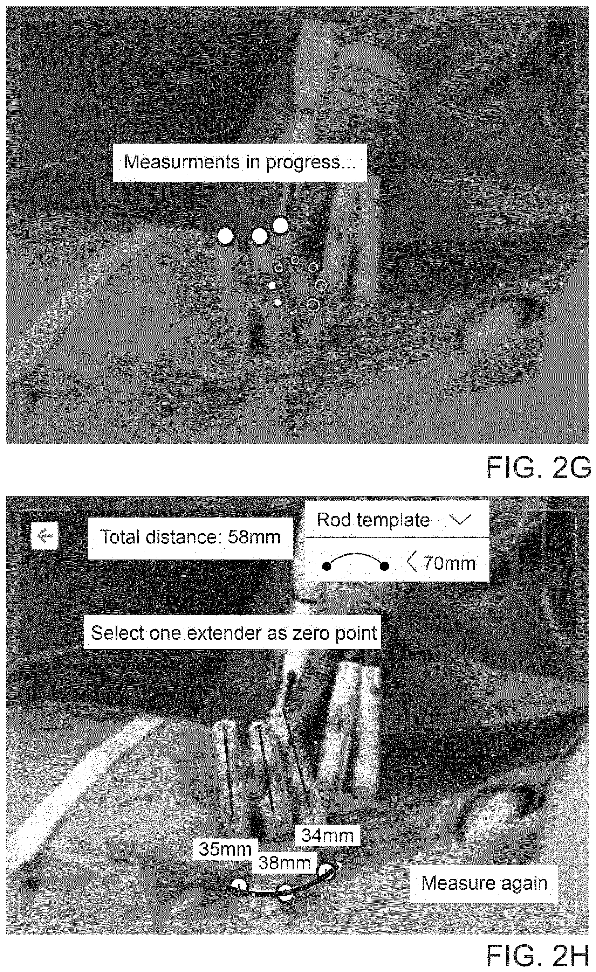

It is also possible that a list of coordinate or other descriptive data of a plurality of different pre-bent rod templates RT is prestored in a data set or structure, for example in a memory of the data processing device 100 or at a server that is accessible by data processing device 100, and this data set is then compared for a best fit with the geometry of the rod attachment locations, for example including the determined attachment center points AP and/or direction of the center axis DCA. Thereby, one or more rod templates can be identified for presentation to surgeon or operator O. The performance of step C30 can be also be displayed to surgeon or operator O on the data processing device 100, for example by a progress bar or circle, animated waiting symbol, as exemplarily illustrated in FIG. 2G.

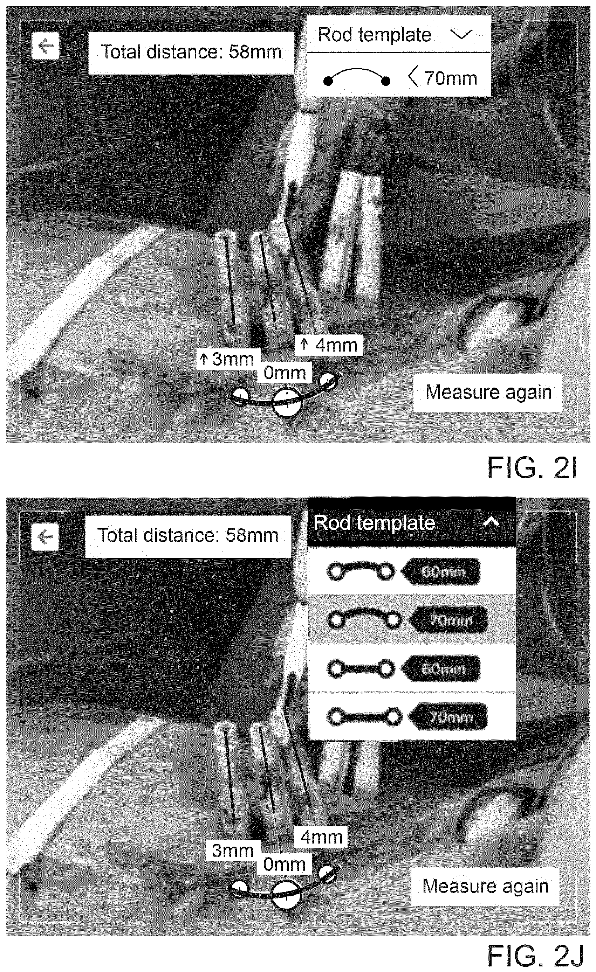

Next, the method 200 can continued to step D40 where different information related to the rod templates RT, attachment center points AP, direction of the center axis DCA can be displayed, and user interface related to this information can be displayed on the GUI, for changing and visualizing different parameters. Exemplary screen shots are provided with FIGs. 2H and 2I. This can be done while the live video feed is still being displayed on display device 120 of data processing device 100, to provide for the augmented reality feature of the application, and thereby providing a visual feedback for the correctness and fit of the rod templates to the pedicle screws PS. As exemplarily shown in FIG. 2I, different information can be displayed as a graphical overlay over the live video feed, including a line for each selected screw extender SE that is a projection of the center axis CA for each screw extender SE, a graphical element that visualizes the calculated attachment center points AP that represent the geometry of the rod attachment locations, placed at a projection location of the attachment center points AP. For this augmented reality aspect, coordinate data of the attachment center points AP can be mapped or projected to the coordinate space of the display. Moreover, a graphic representation of a rod template RT that has been chosen or determined by step C30 can be displayed, including characterizing data, for example but not limited to thickness, length, bending radii, bending patterns. In the variant shown, a bent rod template RT is displayed in box, together with its length in millimeters.

In addition, as shown in FIG. 2I, step D40 can also display the same bent rod template RT of the box, but placed to coincide with at least one of attachment center points AP, to show a graphical representation of rod template RT installed with the three (3) exemplary pedicle screws PS. In the variant shown, rod template RT is displayed such that is center axis coincides with the middle one AP2 of the attachment center points AP, thereby AP2 serving as the zero point or reference point, representing the attachment to screw head SH2 of pedicle screw PS2. Next, for the other attachment center points AP and pedicle screws PS1, PS3, a distance from the rod template RT to the attachment points PS1, PS3, can be displayed, so that the surgeon or operator O can verify by how much the currently chosen rod template RT is fitting or not fitting to the pedicle screws PS1, PS3 that are adjacent to the reference point.

For example, assuming that rod template RT is straight, a geometric calculation in the three-dimensional (3D) space can done to determine a distance from rod template RT, being a straight line, to attachment center points AP1, AP3, by placing two geometric surfaces GS1, GS2 that are perpendicular to the straight line, with attachment center point AP1 lying in one of the surfaces, and attachment center point AP3 lying in the other one of the surfaces. Next, the distances between neighboring attachment center point AP1, AP3 and a point defined by the crossing of the respective surface with the straight line provides for a definition of these two distances that can be displayed. In the case the rod template RT is curved, the same approach can be used, by determining two surfaces GS1, GS2, each being perpendicular to tangential line that is located at the place of intersection of the respective surface GS1, GS2, with attachment center points AP1, AP3 also lying within one of the surfaces GS1, GS2. Thereby, a distance from attachment center points AP1, AP3 to rod template RT can be determined. As exemplarily illustrated in FIGs. 2I, 1J, 2K, these distances can be displayed in millimeters, inside a box for highlighting or for easy reading, associated with a center line CA that is indicative of the longitudinal extension of the screw extender SE, and an arrow, pointer, or other direction-indicating graphical element can be associated with the distance value to indicate a direction of the offset distance from the rod template RT. In case the distances are very small, the indicator can help to identify an offset distance direction. These distance values can be displayed with the graphical user interface, or can also be displayed as a movable text screen with a graphical association with each one of the screw extender SE and pedicle screw PS assemblies, for example with each center line CA that is displayed for screw extender SE.

In the variant shown of FIG. 2I, the reference point or location for the measurements of offset can be changed, for example with a step U55, to determine a different zero or reference point. For example, with step U55, operator or surgeon O can chose a different reference or zero point, by simply touching, pressing or otherwise selecting a graphical element that represents one of the attachment center point AP1, AP2, AP3 with the GUI, and thereby reset the zero or reference point, and the offset values can be recalculated for the new reference point. As another example, the user can select one of the screw extenders SE1, SE2, SE3 as one of the zero or reference points, as exemplarily illustrated in FIG. 2H. Also, the recalculation of all the offset values can be automatic, or can be done upon a confirmation or request by operator or surgeon O, by pressing or touching a button, as shown as the virtual button "measure again" as exemplarily shown in FIGs. 2H, 2I, and 2J.

FIG. 2N shows a variant of the screen that can result from step D40, where an exemplary three (3) different attachments points AP1, AP2, AP3 are shown, and as a function of their distance from the selected and placed rod template RT, a visual feedback is provided to operator or surgeon O with respect to an offset of an attachment point AP1 to AP3 relative to the placed rod template RT. For example, attachment AP3 is shown to be farthest of rod template RT, with a calculated offset distance of about 8mm, and attachment point AP3 is thereby highlighted in a red color, for example a red dot, or other type of highlighting that indicates that the chosen rod template RT is not suitable for placement and attachment to the corresponding pedicle screw PS3. In contrast, attachment point AP2 is shown to be located at the rod template RT, or in an acceptable close range to rod template RT, and therefore can be highlighted in a green color, for example a green dot, or other type of highlighting. The offset has been measures as 0 mm. This indicates that the selected rod template RT would have been suitable for placement at this specific attachment point AP2 Analogously, attachment point AP1 is can be highlighted in an orange color, indicating a not ideal but somewhat suitable position, having an offset distance of 4 mm, as shown in FIG. 2N. In this respect, an increasing distance of an attachment point AP from a rod template RT that has been placed to be connected to one of the attachment points AP1, AP2, AP3, can be indicated with a coloring, or other type of visual feedback. In the illustrated variant of FIG. 2N, a heatmap coloring scheme is used, with the color green representing a good match of one of the AP to the rod template RT, changing from green to orange to red color of a bad match of rod template, for example offset values that are outside of a range for which a rod could be bent to.

Moreover, method 200 can perform a step D50 for displaying a list LL of rod templates RT that can be selected by operator or surgeon O, for visualizing at the surgical incision SI with the live video feed, to allow operator or surgeon O a visual inspection of the rod placement by augmented reality. For example, this step can display a list of rod templates RT that were found based on step C30 where rod templates RT were calculated, for example the ones with the best match for the geometry of the rod attachment locations, or a list of rod templates RT from a pre-stored selection. With the displayed list LL, data processing device 100 is configured to allow operator or surgeon O to graphically select one of the rod templates RT with a step U50, and thereafter, the selected rod template RT can be displayed to be virtually connected or placed to at least one of the attachment center points AP, as shown in FIG. 2J, with step D40. Also, upon selection and virtual placement of the rod template RD to the pedicle screws PS, the offset values can be calculated and displayed.

In this respect, it is also possible that a step D55 is performed, where a selected one of the rod templates RT is displayed as a graphical element at a one-to-one (1: 1) scale of the display screen or graphical user interface GUI. This could be done with two 1: 1 views, for example a sagittal or longitudinal plane view and a coronal or frontal plane view, as an aid for user or operator O to manufacture a corresponding rod. If the rod template RT does not fit into the screen for being too long, for example longer than the screen of a typical tablet, it is possible to preserve the 1:1 view scale, but that a scrolling option is used on the GUI.

In an optional step of method 200, a hint can be provided to surgeon or operator O on how to place or adjust another pedicle screw PS, for example a fourth pedicle screw PS4 when referenced to the image of the surgical scenery shown in FIGs. 2D to 2K, to match coordinates of a selected and placed rod template RT. For example, a graphical element can be displayed that extends from the chosen rod template RT and having a graphical element that illustrates a potential location of attachment location of a next pedicle screw PS4. For example, with reference to FIG. 2J, where a curved rod template RT is shown that is placed at the attachment center points AP1 to AP3 of three different pedicle screws PS1 to PS3, a linear graphical element or a triangular graphical element can be displayed, showing a dot, cross, or other graphical element for indicating a next attachment location for a potential pedicle screw PS4. The triangular graphical element could have a corner at the end of the rod template RT, to indicate different possibilities of attachment of pedicle screw PS4.

In another optional step of the method 200, it is possible that specific screw extender SE and pedicle screw PS assemblies are selected, to compare their position pre-correction, during the corrective operation, and post-correction, to gather data on the change of geometric position relative to each other, for example by calculating, displaying, and processing the different attachment center points AP1-AP3 pre-correction, during the corrective operation, and post-correction, as further described below.

Next, in a step C60, based on a rod template RT that has been selected by operator or surgeon O with step U50, rod template data RTD from a selected rod template RT can be processed to generate CAD data, or other data that can characterize the rod R that would result from the rod template RT, that can be used to manufacture a physical fixation rod R, and the CAD data can be sent to a rod bending machine, or another type of rod processing device for manufacturing an actual physical rod manufactured with a step F10. It is possible that the rod template data RTD is provided by step U20, or indirectly after or simultaneously while being displayed in a one-to-one representation with step D55. In this step, data for manufacturing a rod R based on the RTD from step C30 and selected by operator or surgeon O in step U50. Next, the geometric data for the selected rod can be extracted from the RFT data, and can be converted to a different data format, for example a CAD data format standard, such as but not limited to STEP, IGES, Parasolid, STL, VRML, X3D, DXF, COLLADA. For example, at least one data set for one rod from the RDT can be transmitted to a rod bending or processing machine, for example a rod bending device as described in U.S. Patent No. 6,755,064, U.S. Patent No. 10.405,908, or as described in U.S. Patent Publication No. 2005/0262911, these references herewith incorporated by reference in its entirety.

Another optional step of method 200 is a step C70 for calculation an estimation of pose data information PDI_V of each vertebrae V that is attached to pedicle screw PS, and an optional display step D70 to display graphical primitives on the live video feed or displayed images of each vertebrae V for providing a live video feedback in augmented reality to show an estimated or calculated positioning of the actual vertebrae V of the spinal columns SC, and another optional step C75 of calculating spine curvature data SCD or other spine-characterizing parameters or parametrizations PAR of the spinal column SC, for example but not limited to Cobb angle, Sagittal angle, and other parameters of the spine, to thereby calculate an estimation of the spine curvature of the living being L that is under surgery, without the need of intrusive medical imaging, for example X-ray imaging. With the step of C10 of detecting different screw extenders SE, and the provision of pose data information PDI of each detected screw extender SE, pose data information PDI_V of the vertebrae V can at least be estimated, even if the spinal column SC is not visible in the images of the live video feed. As usually two pedicle screw pair PS are attached to each vertebrae V, this step allows to calculate an estimation of the position, based on two different pose data information PDI of two different screw extenders SE, for example two adjacently arranged screw extenders SE1, SE4 that are both attached to the same vertebra V, as shown in FIG. 1D.

While the exact geometric relationship between screw extender SE and vertebrae V may not be known, there is a probability range that can be used for an approximate estimation, where two (2) pose data information PDI data sets for the two (2) screw extenders SE attached to one vertebra V can be used to provide for an estimated pose PDI_V for each vertebrae V, for example by using mean value of the two screw extender poses PDI. Moreover, based on historic data of geometric relationships between the position or pose of the screw extenders SE that have a fixed location relative to the screw extenders SE, and the position or pose of vertebra V, a knowledge database can be generated to use the most likely position a vertebra of the spinal column SC will take, given the detected PDI of the two screw extenders attached thereto. For example, for step C70, it can be assumed for calculation and estimation purposes that each pair of pedicle screws PS have an ideal, predetermined placement into the vertebrae V for a given vertebrae V, and it could be assumed that the attachment position of pedicle screw PS in terms of a drilling hole center axis, in terms of position and orientation, has been chosen to be at such ideal, predetermined placement position, based on a normative size of a vertebrae V. Upon detection of pose data information PDI from step S10 for pair of screw extenders SE, and the assumption that pairs screw extenders SE, via pedicle screws PS, are attached to an approximation of such ideal position for the pair of screw extenders SE, the position and orientation of the corresponding vertebrae V can be approximated, and pose data information of a corresponding vertebrae V can be calculated by a geometric transformation of the coordinates to obtain pose data information PDI_V.

Steps C70 and C75 can be based on an estimation, calculation, or determination using the knowledge database, the knowledge database having historic information on a correspondence or mapping between attachment points AP of different pedicle screws PS or pose data information PDI of different screw extenders SE, and a position and orientation information of a corresponding vertebrae as PDI_V, spine curvature data SCD, spine-characterizing parameters PAR, or a combination thereof. Thereby, it is possible that an artificial intelligence network is created or established, for example a convolutional neural network (CNN), decision forest, or other type of network, that has been trained with knowledge database to determine pose data information PDI_V of vertebrae, spine curvature data SCD, spine-characterizing parameters PAR including Cobb angles and Sagittal angles, or a combination thereof, from the detected attachment points AP, pose data information PDI, or a combination thereof. As the pose data information PDI and the attachment points AP have a deterministic and calculable geometric relationship, PDI_V, SCD, or PAR can be directly determined from PDI of the detected screw extenders SE. However, in a variant, it is also possible that the calculation of pose data information PDI_V of vertebrae, or the spine curvature data SCD is omitted, and that based on either attachment points AP of different pedicle screws PS, pose data information PDI of different screw extenders SE, or both, the spine-characterizing parameters PAR are directly calculated or estimated, without calculating any pose or other type of positioning or curvature data PDI_V, SCD of the spine itself, as ultimately the user or operator O is particularly interested in these parameters PAR for the spinal correction surgery, for determining the spinal correction.

Also, based on the thus obtained estimated pose data information PDI_V with step C70, step C75 can be performed of calculating spine curvature data SCD to thereby calculate an estimation of the spine curvature of the living being L that is under surgery. This can be again based on a knowledge database, and can take into account patient-specific parameters and values, for example based on age, weight, height, of the patient, a probable spine curve can be calculated as spine curvature data SCD. Also, step C75 can also calculate the spine-characterizing parameters PAR based on the pose data information PDI_V form step C70 of the vertebrae V, or based on pose data information PDI of the screw extenders SE of step C10, or based on both the PDI and PDI _V from steps C10, C70, based on typical algorithms for such determination, for example but not limited to geometric transformations based on vector representations of the PDI, PDI_V in the Euclidian coordinate space. This data can be used for an automated spinal column or vertebral column rectification or correction devices or systems, to at least partially correct a curvature of the spine of the living being by an automated process. The spinal column rectification system could be in the form of a surgical bed that has motorized actuators, a robotic device, or a pillow having expandable chambers, for example, for the automated spinal correction, the systems as described in Chinese Patent Applications CN 108 143 582 or CN 110 279 554, or similar machines, could be used.

With these aspects, it is possible that the method 200 can calculate different pose and positional information of the spine or vertebral column of the living being L. For example, it is possible to calculate different types of spine-characterizing parameters or parametrizations PAR, for example but not limited to the sagittal alignment or the lumbar lordosis of the lumbar spine, including but not limited to the parameters of lordosis tilt angle, global lordosis, sacral slope, lordosis distribution index, apex position of lumbar spine, upper arc angle, the relative spino-pelvic alignment, the sagittal alignment of the thoracic spine or the cervical spine, kyphosis including parameters such as the Cobb angle, sagittal balance, and other parameters. Also, it is possible to calculate different geometric parameters related to a spine or vertebral column suffering of cyphosis.

For example, FIG. 3A shows a schematic and simplified representation of a spine or vertebral column with seven (7) exemplary vertebrae V1-V7, for which for each vertebrae V, two attachment point pairs AP1.1 and AP1.2 have been determined with the step C20 of calculating . Based on the set of attachment points AP that have been calculated, in the variant shown seven (7) pairs of attachment points APn.1 and APn.2, for this example n being from 1 to seven (7), different parameters related to the pose and orientation of the spine or vertebral column can be calculated in additional steps to the method.

For example, with step C70, pose data information PDI_V for each vertebrae V, that can include a three-dimensional (3D) position and orientation information VP1 to VP7, can be calculated for each vertebrae V1 to V7, based on the geometric position data from the pairs of attachment points APn.1 and APn.2 that are associated with each vertebra V. As two different geometric points AP are available for each vertebra V when two pedicle screws PS with screw extenders SE are attached to each vertebrae V, and the exact position of each AP relative to the vertebra is not 100% defined, an average value or geometric middle position of both attachment points APn.1 and APn.2 can be used to calculate the associated VPn for each vertebrae with a more precise position. This calculation can further take statistics into account, for example based on historic data and statistical variation of the attachment point positions AP, and as explained above, a trained artificial network can be used. The three-dimensional (3D) position and orientation information VP1 to VP7 for each vertebrae can also be used for displaying graphical primitives of each vertebrae to the live video feed, as explained above with respect tom the display step U70.

Next, with another step C75 of calculating spine curvature data SCD, based on pose data information PDI_V for each vertebrae V, for example the calculated 3D position and orientation information VP1 to VP7 for each vertebrae V1-V7, a geometric model or a coordinate data of the spine curve can be calculated. For example, spine curvature data SCD can be a curve that has been determined by curve fitting with the geometric points VP1 to VP7, or by characterizing it with a series of geometric positions in the 3D space. However, other data or parameters related to the spine and the vertebrae V1-V7 can be calculated in this step C75. For example, it is possible to calculate the distances between each vertebrae, such as D12, D23, D34, D45, D56, D67, for example based on the distance between geometric points of adjacent vertebrae, it is also possible to calculate an angle of orientation β between adjacent vertebrae V, for example an angle of orientation of two adjacent vertebrae V when viewed from different directions, for example when viewed from the back, when viewed from the front, or when viewed from either side.

Also, with step C75, it is also possible that spine-characterizing parameters or parametrizations PAR are calculated, as explained above. Generally, based on the pose data information PDI_V of each vertebrae V, for example including the calculated 3D position and orientation information VP1 to VP7, different geometric and orientational parameters PAR of the spine or vertebral column can be calculated so that they can be stored, displayed, archived, and reviewed by the surgeon or operator O. As an example, for different types of spine surgeries, spine-characterizing parameters PAR such as but not limited to lordosis tilt angle, global lordosis, sacral slope, lordosis distribution index, apex position of lumbar spine, upper arc angle, relative spino-pelvic alignment, the sagittal alignment of the thoracic spine or the cervical spine, kyphosis including parameters such as the Cobb angle, sagittal balance, and other parameters can be calculated. As another example, based on pose data information PDI_V of each vertebrae V, that can include data on an orientation of each vertebrae V, it is possible to calculate a rotational orientation of adjacently located vertebrae towards each other.

It is also possible that the graphical user interface GUI is configured such that surgeon or operator O can select two vertebrae, for example by clicking on or otherwise selecting the graphical primitives on the display, and thereafter different parameters relative to these two selected vertebrae can be displayed, for example their distance, their rotational orientation with respect to each other, and their pose information, for example to compare their angular orientation.

A display step D70 can be performed, where graphical primitives can be displayed that represent the different vertebrae V, the spine curvature as a line or a curved graphical element, overlaid with the live video feed or the direct view of the surgery with a head-up display, for example based on the spine curvature data SCD that has been calculated by the step C75. Also, in the display step D70, a corrected spine curve CSC can be displayed, and all the different calculated parameters that characterize the spine. In the variant of FIG. 3A, this could be a straight line, as the ideal spine curve seen from the back view would be straight.

According to another aspect, with method 200, it is possible to take measurements of the spine or vertebral column via the screw extenders SE at different time moments during the surgery. For example, surgeon or operator O can first capture and detect the screw extenders SE with step U30, C10, and thereafter the different parameters can be calculated based on steps C20, C40, C70, C75. After choosing and placing a rod R, the user or operator O can insert the rod to the open slit of the screw extenders SE, and then engage in the rod reduction of the rod R such that the rod R moves down into the grooves of the screw extender SE, to place the rod R inside the U-shaped grooves of the screw heads and can be held by a set screw of each pedicle screw PS. During the reduction process, rod R will then force vertebrae into a new position. This will lead to a correction or change of the spinal curve, to a new arrangement and a new spine curvature data SCD once measured, for example a coronal, sagittal, and axial correction. At this stage, or at any other time moment during the reduction process, before removing the screw extenders SE from the pedicle screws PS, the operator or surgeon O can again engage in steps U30, C10 to re-detect all the screw extenders SE, and to re-determine attachment points AP, pose data information PDI_V of each vertebrae V, for example including the calculated 3D position and orientation information VP1 to VP7.

Thereafter, it is possible that different parameters and data are displayed in a displaying step D80, for example by the use of a graphical user interface (GUI), to show the correction or changes before and after the attachment of the rod, thereby using spine curvature data SCD that has been determined pre- and post-correction, based on a repetition of step C10, C70, and C75. Step D80 can display different spinal parameters PAR or spine curvature data SCD pre-surgery, post-surgery, or both, for example as a comparative representation, for example with two table rows or columns of different SCD or PAR pre-surgery and post-surgery. This allows operator O to visually compare the data on a GUI or other representation on the screen. If the correction is insufficient or outside a preferable range, rod R that is in connection with the screw heads SH can be removed or unlocked and a rod R with a different curvature or shape can be placed into the screw heads SH. A curvature or shape of rod R can be changed by an instrument placed on the set screw or on the screw extender SE, before again tightening the rod R by the set screw to the screw head SH. For example, it would be possible to display and visualize different spine-characterizing parameters PAR with the graphical user interface GUI, to compare data of the pre- and post-rod placement, for example but not limited to the most pertinent spine characterizing parameters PAR including Cobb angle, sagittal angle, lordosis tilt angle.

It is also possible that not only the screw extender SE is used for the detection and tracking in steps U30, C10, but also a tool SD that is operatively attached to screw extender SE SC, as illustrated in FIG. 3B. For example, tool SD could be a set screw driver or rod reduction tool for the rod reduction, or a screw driver for threadably engaging the bone anchors of the pedicle screws PS with the vertebrae. Tool SD thereby can fix the axis between the screw extender SE and bone anchor BA of pedicle screw PS, to have a defined orientational relationship. For this purpose, the shape of tool SD can be tracked and detected, or tool SD can also be equipped with optical markers OM, in the variant shown in FIG. 3B two optical markers OM are placed on the top and the bottom of handle of tool SD.

By visualizing changes to the spine or vertebral column before, during and after the rod correction, operator or surgeon O to see directly how much impact he will have on the correction of the spine through the above described measurements and displaying. The rod template RT that has been determined as suitable can be made as a real physical embodiment that will be then placed inside living being L. Based on data of the rod template RT, and the spine pose information, and location of the attachment points AP, it is possible to calculate a spine pose that most likely will result from the chosen rod template RT, before the correction has been made, in other words, before the rod has been attached to the pedicle screws PS.

Also, with a database that can be accessed from different devices 100 that are recording the surgery, training data for future surgeries and deep learning by different type of artificial intelligence (AI) can be created, for example for training a convolutional neural network. For example, for each surgery, video data and the calculated and detected metadata, including the pose information of screw extenders, attachment points AP, rod templates, vertebrae positions can be stored in the database, indexed, and used as training data and archiving in the database.

Under certain circumstances, the angular orientation between bone anchor BA and screw head SH of pedicle screw PS is not fixed, but is limited to a certain angular range, for example by the use of a multi-axial or poly-axial pedicle screw PS having a certain angular orientational range, for example ±27°, or other angular range. In such case, because the screw extender SE is attached to the screw head SH of the pedicle screw PS, the orientation of screw extender SE to bone anchor BA may not be known or visible. As the method 200 can rely on the position and orientation of the screw extender SE to calculate the attachment points AP of the screw head SH, and thereafter the pose data information PDI_V of each vertebrae V, for example to calculate VP1 to VP7, given that the orientation between screw extender SE or screw head SH versus bone anchor BA is not known, and may not be viewable from outside of the surgical incision SI, the calculation of the pose data information PDI_V of the spine can have a relatively high error margin, due to this uncertainty. Under such circumstances, the operator or surgeon O can be instructed to move all screw extenders SE such that they move to the end of the angular range, such that articulating joint formed between screw head SH and bone anchor BA is at a maximal angular point, and thereby the orientational relationship between screw extenders SE and screw head SH and bone anchor BA is fixed and known to a certain degree.

For example, as shown in FIG. 3C, all three (3) exemplary visualized screw extenders SE have been moved to the same direction, to be tilted by the maximal orientational angle of 27° of the poly-axial or multi-axial pedicle screw PS, in the variant shown along a direction of extension of the spine or vertebral column. This step can be instructed to the operator or surgeon O in a step D25 of method 200, by a graphical user interface or other type of instruction, for example by a voice instruction, animation, etc. before operator or surgeon O engages in the step U30 of scanning the surgical incision SI and the screw extenders SE. The instructions could include the display of an arrow or pointer to shown on the live video stream to show the direction of movement to place the screw extenders SE to the outer angular position relative to screw head SH. In a variant, it is possible that tool is used that is inserted into screw extender SE, the tool engaging with bone anchor BA, to provide for a fixed angular relationship between a screw extenders SE and the bone anchor BA, to provide for a temporary mono-axiality of the pedicle screw PS for the measurement and calculations with step C10, C20, C70. This tool could be the screw driver SD itself that can engage through screw extender SE into a part of bone anchor BA, as exemplarily shown in FIG. 3B, for example engaging with a torque driving mechanism of bone anchor BA, or other element of bone anchor BA, to thereby reorient screw head SH to the same axis of extension of the bone anchor BA, thereby being in a orientation of the mono-axial screw configuration. It is also possible that such tool is attached to bone anchor BA for orientation purposes of screw head SH without the use of the screw extender SE, or when the screw extenders SE are removed. Also, it is possible that such tool is equipped with optical markers OM for detection efficiency, as shown in FIG. 3B.

Method 200 is not limited to a performance with a portable data processing device 100, but can also be performed with a non-portable system, for example a multi-camera system with fixedly installed cameras, data processing device or server, and an interactive screen. In such variant, it is possible to use a plurality of cameras that provide for different viewing angles of the surgical incision SI, thereby providing for image data for three-dimensional determinations, and that the live video feed and GUI is displayed on a display screen placed in the surgical operation room. Depending on whether a camera view is obstructed, an algorithm could be operated on data processing device that can switch the camera view. Also, instead of a touch screen, another type of input device could be used, for example a mouse, a laser pointer with corresponding screen, or other input device that can read motions or indications of hand of operator or surgeon O.

As another embodiment, it is also possible that the data processing device 100 includes wearable augmented reality (AR) glasses, a head-mounted display having a transparent or semi-transparent display screen, or a head-up display (HUD), the glasses or display also including a camera for capturing the images sequences for tracking and detection of the screw extenders. For example, a system as described in U.S. Patent No. 10,854,098 could be used, this patent herewith incorporated by reference in its entirety. This allows to provide for a see-through-type augmented reality system, and it may not be necessary to display the live video feed of step D10, as the live video feed is the direct view through the transparent display screen. The graphical elements, such as the graphical primitives GP for screw extenders SE, selector elements SF, text boxes, rod templates RT, and other elements of the graphical user interface, can still be displayed on the transparent display screen.

According to another aspect, it is possible that different radio-opaque markers ROM are placed on the skin of the living being L that is under surgery, or other types of markers that can be detected by X-ray or CT scanning, or detectable by other types of medical imaging. For example, the ROM markers can represent a QR code or other type of optical code. This allows to make intra-operational imaging with the markers ROM in position to make a connection between the intra-operational patient images, for example by X-ray with a C-arm, or CT-scan through O-arm or 3D C-arm, to determine positions and orientations of the bone anchors BA and screw heads SH, and thereafter these positions can be matched to pose information of the screw extenders SE, either by 3D shape matching with the image data from the image sensor, or by QR code matching.

As another aspect of the herein presented method, it is possible to provide for guidance to the operator or surgeon O for the positioning of the screw extender SE to facilitate the rod insertion. Sometimes it can be difficult to insert the rod percutaneously in long constructs with a relatively high number of screw extenders SE and pedicle screws PS, as the pedicle screws PS may not all be aligned. For example, one might be more laterally placed or another more medially. However, based on the known spatial position of each pedicle screw PS, for example by the attachment center points AP, surgeon or operator O can tilt the different screw extenders SE to the opposite side to which the screw is misplaced or misaligned relative to the other. For a laterally positioned pedicle screw PS, surgeon or operator O can tilt the screw extender SE medially, and tilt the screw extenders SE laterally for the pedicle screw PS positioned medially. With this repositioning of the screw extenders SE, and the consequential reorientation of the screw heads SH, a better alignment of all slots or openings of the screw extenders SE can be provided, thereby facilitating the insertion of the rod R.

Another aspect of the present invention includes a method 500 for scanning, displaying, and verifying a bent spinal rod R for attachment to attachment points AP. An exemplary flowchart of method 500 exemplarily shown in FIG. 4, where a real spinal rod R can be scanned and visualized, for example by the use of a live video of the surgical incision with a live video feed using augmented reality, with spinal rod R as a template RT in context of the different attachment points AP defined by pedicle screws PS that are attached to the spinal column SC.

With method 500, a spinal rod R that has been bent by surgeon, operator, or user O, can be scanned, filmed, or image taken that are subject to a calculation step by data processing device 100 or 320, for example with a scanning step U100. It is also possible that the real rod R has been manufactured by a step F10 as explained above, for example based on a chosen rod template RT. For example, this step can also be aided or complemented by three-dimensional data or depth data from a time-of-flight type sensor, for example a Lidar sensor. Next, a step C110 can be performed, where the captured image data, for example a video sequences or image sequence with views from different angles of the rod R, or based on three-dimensional or depth data, a geometric data that represents rod R can be calculated, as a rod data set RD. Next, with a step D40, a projection or a rendering of the real rod R as a rod template can be done, and the read rod template RRT can be displayed, and selected, for example to be attached to one of the attachment points AP as shown in FIGs. 2H, 2I, 2J, 2K, and 2N. With a step U55 as explained above, the reference attachment point AP as the zero-offset point can be changed, so that different placements of the real rod template RRT can be visually verified by operator O. Next, a step C120 can be performed, where based on the real rod template RRT, and the initially suggested or chosen reference attachment point AP, the remaining attachment points AP are moved or corrected to be coinciding with the real rod template RRT.

With step C120, an approximation of the corrected spinal curve SC can be calculated, based on the original data of the spinal curve SCD in the pre-correction state that originates from step C10 and C75, based on a proposed rod R and the rod data set RD for the scanned and calculated real rod template RRT from step C110, and from a step C120 where the corrected location of attachment points AP can be calculated, based on an initially proposed or chosen reference position of real rod template RRT, for example a placement of RRT to coincide with a position with a chosen one of the pre-corrected attachment points AP. Thereby, with step C120, a new data set of the virtually moved attachment points AP will be calculated that would be departed to the attachment points AP, if the real spinal rod R would be attached to the pedicle screws PS. Next, the method 500 could also include a step of calculating pose data information PDI_V of all the vertebrae V involved, based on the newly calculated virtual attachment points, with a step C70 as explained above, and a step of displaying graphical primitives for vertebrae V or a rendering of the spinal column SC with a step D70 as explained above, to visualize the virtually corrected spinal column SC.

The displaying with step D70 would show spinal column SC as virtual or augmented reality graphical primitives, that would be based on the bent rod R, so that the surgeon, operator, or user O can verify virtually the effect the bent rod R would have on the spinal column SC. This allows the verify if bent rod R will have the desired effects before the need of attaching the rod R to the attachment points AP of the pedicle screws PS. Also, steps C75 and D80 could be performed, where the spinal column curve data SCD and spinal parameters are calculated and thereafter displayed with step D80. Step D80 could also include the displaying of spinal column curve data SCD and spinal parameters per-correction, based on a previously performed step C75 and D80 for the pre-correction location of the attachment points AP. The displaying of the real spinal curvature data SCD pre-correction and the virtual spinal curvature data SCD would allow an operator O to verify whether the bend rod R would have or at least approximate the desired corrective effect to the spinal column SC.

According to another aspect of the present invention, a method 600 is provided for determining different type of information that characterizes the spinal column SC before the placement and anchoring of the pedicle screws PS to a respective vertebrae is made, as exemplarily and schematically illustrated with the flow chart of FIG. 5. Therefore, method 600 allows to calculate different spinal data and parameters before performing any spinal correction by a fixation rod R attached to pedicle screws PS, for example by determining spinal parameters PAR, or spinal curvature data SCD, and pose data information of different vertebrae PDI_V, by first detecting different pedicle markers PM that can be inserted to or otherwise attached to different vertebrae V, via a guide wire or another equivalent device. This allows to perform a step of defining or suggesting a fixation rod R for the spinal correction surgery even before the pedicle screws PS are attached to the vertebrae V that would define the attachment points AP for fixation rod R.

For example, as described in U.S. Patent Publication No. 2021/0169506, this reference herewith incorporated by reference in its entirety, a pedicle marker PM is described that can be attached to the guide wire GW, such as but not limited to a Kirschner wire, K-wire, guide pin, Schanz pin, Denham pin, Steinmann pin, guide rods, and guide shaft, that are inserted and placed into the initial drill holes DH or bores via the surgical incision SI, the drill holes having been drilled into the different vertebrae V of a spinal column SC, and pedicle markers can be attached to the guide wire. Guide wires GW can be placed in each of the drill holes DH to guide pedicle screw PS into the drill hole or bore for insertion into the pedicle or vertebra V. The bone anchor BA of pedicle screw PS typically includes a through-bore through which the guide wire is passed allowing pedicle screw PS to be guided to the drill hole or bore formed in the pedicle. Pedicle markers PM can be used to be attached to the guide wires or their equivalent, for example but not limited to the ones described in U.S. Patent Publication No. 2021/0169506, allowing to facilitate the insertion and placement of pedicel screws PS to the guide wires, and also for facilitating the surgery for surgeon or operator O by helping the placement of guide wires GW and removal of the guide wires from the drill holes.

FIG. 6 shows an exemplary and simplified cross-sectional representation of a vertebra V with two drill holes DH1, DH2 having been drilled or otherwise made into vertebra V, two guide wires GW1, GW2 placed into drill holes DH1, DH2, respectively, and two pedicle markers PM1, PM2 attached to guide wires GW1, GW2, respectively, with optical markers OM provided with a removable or fixedly attached optical marker part 50, optical markers OM usable for a robust detection of the pedicle markers PM1, PM2, guide wires GW1, GW2, or both, by the use of computer image data processing with tracking algorithms. In the variant shown, optical marker part 50 is exemplarily made as a removable cap, clip, tube, clamp, flag, tab, or other device having two flat surfaces for placing an optical marker OM each for redundancy, similarly to the device 50 shown in FIG. 1E, but it is also possible that optical markers OM are directly arranged on pedicle markers PM, for example as an etched pattern, printed pattern, stamped or embossed pattern, machined three-dimensional surface or structure, or other marking to pedicle marker PM. Also, optical markers OM can also be placed directly to guide wire GW with or without the optical marker OM on pedicle markers PM, for example but not limited to a tab, flag, longitudinal code along a shaft that forms guide wire GW, three-dimensional structure that directly represents a code.

Method 600 has some similar aspects as the method 200 described above, but instead of detecting screw extenders SE with or without the use of optical markers OM in a step C10, steps are performed that can detect pedicle markers PM, to determine information that characterizes the spinal column SC. Steps U10, D10, U20, and D20 can be substantially the same as of method 200, for providing a live video feed on the display 120, providing for a GUI for user operation, and for entering calibration information. A step U230 can be performed where surgeon or operator O scans surgical incision SI with an image capturing device 110 of data processing device 100, with the goal to capture images of the different pedicle markers PM. Next, data processing device 100 perform a step C210 to detect pedicle markers PM by image data processing, for example by pedicle markers PM that are equipped with optical markers OM, or by detecting the shape of pedicle markers PM with image shape or pattern recognition without the use of optical markers, or by detecting optical makers OM that are directly attached to the guide wire GW itself, or are an integral part of the guide wires GW. It is also possible that guide wires GW themselves are detected in this step. The resulting information of step C210 can be pose data information PDI_PM of pedicle markers PM or pose data information of the guide wires GW, or other type of coordinate data that can characterize a position and orientation of the respective guide wires GW. Assuming that two (2) guide wires GW or pedicle markers PM are attached to one vertebra V, this information can be used to determine a position and orientation of individual vertebrae V of spinal column SC.

Thereafter, an optional step D225 can be performed to overlay a graphical primitive on the live video feed, to highlight the pedicle markers PM, the guide wires GW, or both, aiding the surgeon or operator O to select or deselect the pedicle markers PM or guide wires GW of interest for further calculation, with step D230 for showing graphical elements for making the selection, and step U240 receiving input data from surgeon or operator O that actually selects the different pedicle markers PM or guide wires GW that have been detected, analogously to steps D25, D30, U40. Next, an optional step C220 of calculating the geometry can be performed by data processing device 100, where virtual attachment points AP_V can be calculated, being a specific geometric location where fixation rod R will most likely be located with respect to a corresponding pedicle screw PS, the pedicle screw PS not yet being attached or anchored to the vertebrae V. Herein, the attachment points AP_V are considered to be virtual, as no such attachment point AP is currently existing. Thereby, with step C220, an estimate of a geometric location of an attachment point AP can be provided, as virtual attachment points AP_V that can be used to estimate different curvatures or spine parametrizations of the currently operated spinal column SC, and the curvatures or spine parametrizations that will be departed to spinal column SC, if a specific fixation rod R would be placed and attached to these virtual attachment points AP_V, thereby not yet having any direct information on the real attachment points AP. This calculation can be done by the use of artificial intelligence with a trained network using historic data on the locations of the attachment points AP for a given drill hole and a given guide wire GW placed into drill hole from image data, for example based on historic medical imaging data such as but not limited to X-ray images, or by using tables or other prestored information on statistical data of the geometric relationship between positions and orientations of guide wires GW, positions and orientations of pedicle markers PM, and the positions and orientations of pedicle screws PS attached to the vertebrae V, that define the position of the attachment points AP.

Next, a step C270 that is similar to step C70 of method 200 can be performed, this step configured to calculate pose data information PDI_V of each vertebrae V that is in connection with guide wire GW, or guide wire GW and pedicle marker PM, and an optional display step D70 that is analogous to the same step of method 200 can be performed to display graphical primitives on the live video feed or displayed images for each vertebrae V for providing a live video feedback in augmented reality to show an estimated or calculated positioning of the actual vertebrae V of the spinal columns SC projected to the live video feed. Step C270 can use the pose data information PDI_PM of pedicle markers PM or pose data information of the guide wires GW, for two or more vertebrae V, or can also use the data of the pair of virtual attachment points AP_V for two or more vertebrae V from step C220, or both data sets AP_V and PDI_PM.

Also, and another optional step C75 can be performed, analogous to method 20 where spine curvature data SCD or other spine-characterizing parameters or parametrizations PAR of the spinal column SC can be calculated, for example curvature data SCD that geometrically characterizes an approximation of the current spinal curve, and for example but spinal parametrization data such as Cobb angle, Sagittal angle, axial angle, distance between adjacent vertebrae, and other parameters of spinal columns SC, to thereby calculate an estimation of the spine curvature of the living being L that is under surgery, without the need of intrusive medical imaging, for example X-ray imaging, and even before any pedicle screws PS have been placed or anchored. This data SCD and PAR can thereafter be displayed on the display 120 of data processing device 100, to provide a feedback to surgeon or operator O.

As indicated above, with method 600 it is possible to verify a correction that will be departed to spinal column SC by estimation, before any attachment of a pedicle screw PS. For example, after performing method 600 once, surgeon or operator O will have some first estimated information on spinal column SC with data on SCD and PAR that can be displayed with step D80, and can even have a visual feedback of the curvature and position of spinal column with step D270, displaying overlayed primitives projected to the live video feed. Thereby, surgeon or operator O can choose and place a spine cage, fusion device, or other type of intervertebral implant between two exemplary adjacent vertebrae V1, V2 of spinal column SC, and can thereby also choose a type and configuration of the intervertebral implant, for example by choosing a thickness thereof, or by choosing and adjusting a specific angle, for example the sagittal angle, for spinal fusion surgery. The placement of the intervertebral implant can depart certain reorientation and displacement between the position and orientation of two adjacent vertebrae V1, V2, and thereby operator or surgeon O can perform method 600 again, to determine the new values of SCD and PAR of the partially corrected spinal columns SC, based on the placement of the intervertebral implant, but without any placement of pedicle screw pairs PS1, PS2, and without any attachment of fixation rod R.

Based on the newly displayed parameters PAR and values for SCD, or both, step D80 of method 600 can provide for tables, curves, or other type of visualization of the PAR and SCD before the insertion of the intervertebral implant and after the insertion, to provide for comparative data of a first correction to spinal column before any rod R is placed. This allows operator or surgeon O to replace the intervertebral implant with a different one having a different configuration if operator or surgeon O is not satisfied with the new PAR and/or SCD departed by the first intervertebral implant that was calculated by method 600, for example with a different thickness or angle. Also, in a variant, operator or surgeon O can change the parametrization of the intervertebral implant, if the intervertebral implant is configurable type, to change the thickness or distance between the upper and lower bone-engaging faces, or the angle between upper and lower bone-engaging faces. Thereafter, method 600 can be performed again the verify the results of the change of the dimensions and characterization of the intervertebral implant.

Moreover, because method 600 can optionally calculate virtual attachment points AP_V for pairs of potentially placed pedicle screws PS1, PS2 to drill holes DH1, DH2, with a step C220, it is possible to make a determination of a potential rod R by calculating one or more rod templates RT that can be proposed to operator or surgeon O, for example with steps C30, D40, D50, U50, U55, D55, C60 of method 200, to thereby propose and virtually test different proposed rod templates RT for the spinal correction, with one or more rod templates RT with step D50, and the creation of manufacturing data or info with steps D55, C60.

Also, aspects of method 600 and method 200 can also be combined with steps of method 500 where the impact of a rod template RT or rod data RD on a spinal column SC can be virtually tested by calculation, before the physical fixation rod R is actually attached to any pedicle screws PS. For example, after performance of step of selecting a rod template RT, for example with step U50, or with step C30 where a rod template RT is calculated and proposed by method 200, this data can be processed by step C120 where the new attachment points AP are calculated, based on the virtual attachment points AP_V that originated from step C220 of method 600, thereby calculating information on the position and orientation of the vertebrae PDI_V of a virtually corrected spinal column SC, based on a proposed, virtual rod template RT or rod data RD. This aspect is different from method 500, as the data on the attachment points AP is merely virtual, herein referred to as AP_V, because no pedicle screws PS have been yet placed. Steps C70, D70, C75, and D80 can also be performed, to calculate data on the spine curvature SCD and spine parameters PAR (step C75), to display different data on spine curvature SCD and spine parameters PAR (step D80), for example post and pre-correction data, and to display vertebrae V as primitives at their new virtual position and orientation, for example with an augmented reality projection to the live video feed, with step D70.

Once satisfied with the virtual determinations of the rod template RT, a physical fixation rod R can be manufactured, for example with the help of steps D55, C60, or with the help of method 500, and the surgeon or operator O can attach pedicle screws PS to the drill holes DH of the vertebrae V, and thereafter surgeon or operator O can also attach fixation rod R to the pedicle screws PS. With the help of method 200, the spine correction can be verified after placement of pedicle screws PS and fixation rod R. Alternatively, first surgeon or operator O can attach pedicle screws PS to the drill holes DH of the vertebrae V before actually manufacturing rod R, and then method 200 can be performed, to verify the attachment points AP that are now precisely defined by the attachment of the pedicle screws PS, to determine another or corrected rod template RT or rod data RD for a physical fixation rod R. At this stage, method 200 can be performed, to verify the spinal correction that is imparted by physical fixation rod R, during the surgery.

As shown above, the herein described methods 200, 500, and 600, and combinations of the steps of these methods and parts thereof, can be implemented to different types of data processing devices 100, but can also be programmed as computer-readable code that can stored to a non-transitory computer readable medium, for example a data memory device or data storage device of any kind, and the computer-readable code configured to perform the methods 200, 500, 600 or steps thereof, when executed on a data processing device 100, or executed on data processors of other types of computer systems, for example distributed computer systems with network and/or cloud access. For example, it is possible that a tablet-type device is used for the image visualization and image capturing, but the actual calculation steps are performed remotely at a server or personal computer that is in operative connection with the tablet via a network, as a variant of distributed computing.

A method for assisting orthopedic surgery to a spinal column, the method performed with a data processing device, the data processing device including a display device and an image capturing device, the method comprising the steps of:
capturing a sequence of images with the image capturing device such that a field of view of the image capturing device captures images of a plurality of screw extenders, each screw extender holding a pedicle screw, the plurality of screw extenders arranged at a surgical incision of a body of a living being undergoing orthopedic surgery;
providing for a live video feed on the display device by either displaying at least some of images of the captured images or by a direct view with a transparent display device;
detecting the plurality of screw extenders with the data processing device based on the captured sequence of images;
first calculating an orientation and position of the detected plurality of screw extenders;
second calculating a three-dimensional (3D) position of a screw head of each pedicle screw based on the orientation and the position of the first calculating; and
projecting and displaying each calculated 3D position of the plurality of screw heads with a graphical element with a graphical user interface on the display device at a location that corresponds to the location of the screw head projected to a currently provided image of the live video feed.

The method according to the immediately preceding paragraph, further comprising the step of:
displaying a plurality of fixation rod templates as graphical elements on the graphical user interface of the display device, the plurality of fixation rods having different shapes.

The method according to the preceding paragraph (000125), further comprising the steps of:
fitting a curve to points represented by the plurality of 3D positions of the screw heads; and
displaying a template of a fixation rod as a graphical element on the display device, the fixation rod being shaped to at least partially match the fitted curve.

The method according to the preceding paragraph (000126), further comprising the steps of:
graphically selecting one of the plurality of fixation rod templates;
placing a graphical element that represents the selected one of the plurality of fixation rod templates at a location of one of the 3D positions of the screw head.

The method according to the preceding paragraph (000125), further comprising the steps of:
visually highlighting the detected plurality of screw extenders; and
permitting selection or deselection of at least one of the plurality of screw extender with a graphical user interface of the data processing device.

The method according to the preceding paragraph (000125), wherein the data processing device further includes a distance measurement sensor, the method further comprising the step of:
capturing distance information with the distance measurement sensor,
wherein the step of first calculating is further based on the distance information.

The method according to the immediately preceding paragraph, wherein
wherein the distance measurement sensor includes a direct time of flight (dToF) , LiDAR sensor, or a structure light sensor such as FaceID.

The method according to the preceding paragraph (000125), further comprising the steps of:
third calculating pose data information for at least two vertebra based on the orientation and the position of at least one of the detected plurality of screw extenders that are attached to the vertebrae from the step of first calculating; and
projecting and displaying a graphical primitive representing the vertebrae with the graphical user interface on the display device, the graphical primitive being displayed at a location that corresponds to the location of the vertebra projected to a currently provided image of the live video feed.

The method according to the immediately preceding paragraph, further comprising the steps of:
fourth calculating spine curvature data of the spinal column based on the orientation and the position of the detected plurality of screw extenders that are attached to the vertebrae from the step of first calculating; and
fifth calculating spinal parameters of the spinal column based on the orientation and the position of the detected plurality of screw extenders that are attached to the vertebrae from the step of first calculating.
projecting and displaying a graphical primitive representing a curvature of the spinal column with the graphical user interface on the display device, the graphical primitive being displayed at a location that corresponds to the location of the spinal column projected to a currently provided image of the live video feed.

A data processing device configured to assist an orthopedic surgery to a spinal column, the data processing device including a display device, a data processor, and an image capturing device, the data processor configured to:
instruct a capturing of a sequence of images with the image capturing device such that a field of view of the image capturing device captures images of a plurality of screw extenders, each screw extender holding a pedicle screw, the plurality of screw extenders arranged at a surgical incision of a body of a living being undergoing orthopedic surgery;
instruct a displaying at least some of images of the captured images to provide for a live video feed on the display device;
perform a detection algorithm to detect the plurality of screw extenders with the data processing device based on the captured sequence of images;
first calculate an orientation and position of the detected plurality of screw extenders;
second calculate a three-dimensional (3D) position of a screw head of each pedicle screw based on the orientation and the position of the first calculating; and
project and display each calculated 3D position of the plurality of screw heads with a graphical element with a graphical user interface on the display device at a location that corresponds to the location of the screw head projected to a currently displayed image of the live video feed.

A non-transitory computer readable medium, the computer readable medium having computer instruction code recorded thereon, the computer instruction code configured to perform a method for computer-assisting an orthopedic surgery to a spinal column when the computer instructions are executed on a data processing device that is in operative connection to a display device and an image capturing device, the method comprising the steps of:
capturing a sequence of images with the image capturing device such that a field of view of the image capturing device captures images of a plurality of screw extenders, each screw extender holding a pedicle screw, the plurality of screw extenders arranged at a surgical incision of a body of a living being undergoing orthopedic surgery;
providing for a live video feed on the display device by either displaying at least some of images of the captured images or by a direct view with a transparent device;
detecting the plurality of screw extenders with the data processing device based on the captured sequence of images;
first calculating an orientation and position of the detected plurality of screw extenders;
second calculating a three-dimensional (3D) position of a screw head of each pedicle screw based on the orientation and the position of the first calculating; and
projecting and displaying each calculated 3D position of the plurality of screw heads with a graphical element with a graphical user interface on the display device at a location that corresponds to the location of the screw head projected to a currently provided image of the live video feed.

A method for assisting orthopedic surgery to determine a correction of a spinal column based on a fixation rod, the method performed with a data processing device, the method comprising the steps of:
scanning a fixation rod with an image capturing device to obtained scanned data of the fixation rod, the spinal correction rod having been bend for a spinal correction;
first calculating a curvature data of the fixation rod based on the scanned data;
receiving data of locations of attachment points for the fixation rod to the spinal column, the locations of the attachment points having been determined based on positional data of screw heads of pedicle screws that are attached to at least two vertebrae of the spinal column;
second calculating data of corrected locations of the attachment points, the corrected locations of the attachment points being based on a correction that is imparted to the locations of the attachment points when the fixation rod were to be attached to the attachment points of a corrected spinal column, by taking account the curvature data of the fixation rod from the step of first calculating;
third calculating a spinal parameter of the corrected spinal column based on the data of the corrected locations of the attachment points of the corrected spinal column; and
displaying the spinal parameter of the corrected spinal column on a display device.

The method for assisting orthopedic surgery to determine a correction of the spinal column according to the immediately preceding paragraph, further comprising the step of:
fourth calculating pose data information for vertebrae of the corrected spinal column based on the data of the corrected locations of the attachment points of the corrected spinal column.

The method for assisting orthopedic surgery to determine a correction of the spinal column according to the immediately preceding paragraph, further comprising the step of:
displaying a graphical primitive representing the vertebrae with the graphical user interface on the display device, the graphical primitive being displayed at a location that corresponds to the location of the vertebrae.

A method for assisting orthopedic surgery to a spinal column, the method performed with a data processing device, the data processing device including a display device and an image capturing device, the method comprising the steps of:
capturing a sequence of images with the image capturing device such that a field of view of the image capturing device captures images of at least one of a plurality of pedicle markers placed on a plurality of guide wires, respectively, or a plurality of guide wires, the plurality of pedicle markers or the plurality of guide wires arranged at a surgical incision of a body of a living being undergoing orthopedic surgery;
providing for a live video feed on the display device by either displaying at least some of images of the captured images or by a direct view with a transparent display device;
detecting the plurality of pedicle markers or the plurality of guide wires with the data processing device based on the captured sequence of images;
first calculating an orientation and position of the detected plurality of pedicle markers or the detected plurality of guide wires; and
second calculating pose data information for at least two vertebrae based on the orientation and the position of at least one of the detected plurality of pedicle markers or the detected plurality of guide wires that are attached to the vertebra from the step of first calculating.

The method of the immediately preceding paragraph, further comprising the steps of:
third calculating parameters that characterize the spinal column from the pose data information from the step of second calculating; and
displaying the parameters that characterize the spinal column on the display device.

The method of according to the preceding paragraph (000139), further comprising the step of
fourth calculating a virtual three-dimensional (3D) position of a position of a screw head of each pedicle screw based on the orientation and the position of the first calculating; and
projecting and displaying each calculated virtual 3D position of the plurality of screw heads with a graphical element with a graphical user interface on the display device at a location that corresponds to the location of the screw head projected to a currently provided image of the live video feed.

While the invention has been disclosed with reference to certain preferred embodiments, numerous modifications, alterations, and changes to the described embodiments are possible without departing from the sphere and scope of the invention, as defined in the appended claims and their equivalents thereof. Accordingly, it is intended that the invention not be limited to the described embodiments, but that it have the full scope defined by the language of the following claims.

## Claims

1. A data processing device configured to assist an orthopedic surgery to a spinal column, the data processing device including a display device, a data processor, and an image capturing device, the data processor configured to:
instruct scanning of a fixation rod with the image capturing device to obtain scanned data of the fixation rod, the spinal correction rod having been bend for a spinal correction;
first calculate a curvature data of the fixation rod based on the scanned data;
receive data of locations of attachment points for the fixation rod to the spinal column, the locations of the attachment points having been determined based on positional data of screw heads of pedicle screws that are attached to at least two vertebrae of the spinal column;
second calculate data of corrected locations of the attachment points, the corrected locations of the attachment points being based on a correction that is imparted to the locations of the attachment points when the fixation rod were to be attached to the attachment points of a corrected spinal column, by taking account the curvature data of the fixation rod from the first calculation;
third calculate a spinal parameter of the corrected spinal column based on the data of the corrected locations of the attachment points of the corrected spinal column; and
instruct a displaying the spinal parameter of the corrected spinal column on the display device.

2. The data processing device of the previous claim, wherein the data processor is configured to:
fourth calculate pose data information for vertebrae of the corrected spinal column based on the data of the corrected locations of the attachment points of the corrected spinal column.

3. The data processing device of the previous claim, wherein the data processor is configured to instruct a displaying a graphical primitive representing the vertebrae with a graphical user interface on the display device, the graphical primitive being displayed at a location that corresponds to the location of the vertebrae.

4. A non-transitory computer readable medium, the computer readable medium having computer instruction code recorded thereon, the computer instruction code configured to perform a method for computer-assisting an orthopedic surgery to a spinal column when the computer instructions are executed on a data processing device that is in operative connection to a display device and an image capturing device, the method comprising the steps of:
scanning a fixation rod with an image capturing device to obtained scanned data of the fixation rod, the spinal correction rod having been bend for a spinal correction;
first calculating a curvature data of the fixation rod based on the scanned data;
receiving data of locations of attachment points for the fixation rod to the spinal column, the locations of the attachment points having been determined based on positional data of screw heads of pedicle screws that are attached to at least two vertebrae of the spinal column;
second calculating data of corrected locations of the attachment points, the corrected locations of the attachment points being based on a correction that is imparted to the locations of the attachment points when the fixation rod were to be attached to the attachment points of a corrected spinal column, by taking account the curvature data of the fixation rod from the step of first calculating;
third calculating a spinal parameter of the corrected spinal column based on the data of the corrected locations of the attachment points of the corrected spinal column; and
displaying the spinal parameter of the corrected spinal column on a display device.

5. The non-transitory computer readable medium, according to the previous claim, wherein the method further comprises the step of:
fourth calculating pose data information for vertebrae of the corrected spinal column based on the data of the corrected locations of the attachment points of the corrected spinal column.

6. The non-transitory computer readable medium, according to the previous claim, wherein the method further comprises the step of:
displaying a graphical primitive representing the vertebrae with a graphical user interface on the display device, the graphical primitive being displayed at a location that corresponds to the location of the vertebrae.

7. A method for assisting orthopedic surgery to determine a correction of a spinal column based on a fixation rod, the method performed with a data processing device, the method comprising the steps of:
scanning a fixation rod with an image capturing device to obtained scanned data of the fixation rod, the spinal correction rod having been bend for a spinal correction;
first calculating a curvature data of the fixation rod based on the scanned data;
receiving data of locations of attachment points for the fixation rod to the spinal column, the locations of the attachment points having been determined based on positional data of screw heads of pedicle screws that are attached to at least two vertebrae of the spinal column;
second calculating data of corrected locations of the attachment points, the corrected locations of the attachment points being based on a correction that is imparted to the locations of the attachment points when the fixation rod were to be attached to the attachment points of a corrected spinal column, by taking account the curvature data of the fixation rod from the step of first calculating;
third calculating a spinal parameter of the corrected spinal column based on the data of the corrected locations of the attachment points of the corrected spinal column; and
displaying the spinal parameter of the corrected spinal column on a display device.

8. The method for assisting orthopedic surgery to determine a correction of the spinal column according to claim 7, further comprising the step of:
fourth calculating pose data information for vertebrae of the corrected spinal column based on the data of the corrected locations of the attachment points of the corrected spinal column; or
further comprising the step of:
fourth calculating pose data information for vertebrae of the corrected spinal column based on the data of the corrected locations of the attachment points of the corrected spinal column, and
further comprising the step of:
displaying a graphical primitive representing the vertebrae with the graphical user interface on the display device, the graphical primitive being displayed at a location that corresponds to the location of the vertebrae.

9. A data processing device configured to assist an orthopedic surgery to a spinal column, the data processing device including a display device, a data processor, and an image capturing device, the data processor configured to:
instruct capturing of a sequence of images with the image capturing device such that a field of view of the image capturing device captures images of at least one of a plurality of pedicle markers placed on a plurality of guide wires, respectively, or a plurality of guide wires, the plurality of pedicle markers or the plurality of guide wires arranged at a surgical incision of a body of a living being undergoing orthopedic surgery;
instruct providing for a live video feed on the display device by either instructing a displaying of at least some of the images of the captured images or by instructing a direct view with a transparent display device;
detecting the plurality of pedicle markers or the plurality of guide wires based on the captured sequence of images;
first calculate an orientation and position of the detected plurality of pedicle markers or the detected plurality of guide wires; and
second calculate pose data information for at least two vertebrae based on the orientation and the position of at least one of the detected plurality of pedicle markers or the detected plurality of guide wires that are attached to the vertebra from the step of first calculating.

10. The data processing device of the previous claim, wherein the data processor is configured to:
third calculate parameters that characterize the spinal column from the pose data information from the second calculation; and
instruct displaying the parameters that characterize the spinal column on the display device.

11. The data processing device of claim 9 or 10, wherein the data processor is configured to:
fourth calculate a virtual three-dimensional (3D) position of a position of a screw head of each pedicle screw based on the orientation and the position of the first calculation; and
instruct projecting and displaying of each calculated virtual 3D position of the plurality of screw heads with a graphical element with a graphical user interface on the display device at a location that corresponds to the location of the screw head projected to a currently provided image of the live video feed.

12. A non-transitory computer readable medium, the computer readable medium having computer instruction code recorded thereon, the computer instruction code configured to perform a method for computer-assisting an orthopedic surgery to a spinal column when the computer instructions are executed on a data processing device that is in operative connection to a display device and an image capturing device, the method comprising the steps of:
capturing a sequence of images with the image capturing device such that a field of view of the image capturing device captures images of at least one of a plurality of pedicle markers placed on a plurality of guide wires, respectively, or a plurality of guide wires, the plurality of pedicle markers or the plurality of guide wires arranged at a surgical incision of a body of a living being undergoing orthopedic surgery;
providing for a live video feed on the display device by either displaying at least some of images of the captured images or by a direct view with a transparent display device;
detecting the plurality of pedicle markers or the plurality of guide wires with the data processing device based on the captured sequence of images;
first calculating an orientation and position of the detected plurality of pedicle markers or the detected plurality of guide wires; and
second calculating pose data information for at least two vertebrae based on the orientation and the position of at least one of the detected plurality of pedicle markers or the detected plurality of guide wires that are attached to the vertebra from the step of first calculating.

13. The non-transitory computer readable medium of the previous claim, wherein the method further comprises the steps of:
third calculating parameters that characterize the spinal column from the pose data information from the step of second calculating; and
displaying the parameters that characterize the spinal column on the display device.

14. The non-transitory computer readable medium of claim 12 or 13, wherein the method further comprises the steps of:
fourth calculating a virtual three-dimensional (3D) position of a position of a screw head of each pedicle screw based on the orientation and the position of the first calculating; and
projecting and displaying each calculated virtual 3D position of the plurality of screw heads with a graphical element with a graphical user interface on the display device at a location that corresponds to the location of the screw head projected to a currently provided image of the live video feed.

15. A method for assisting orthopedic surgery to a spinal column, the method performed with a data processing device, the data processing device including a display device and an image capturing device, the method comprising the steps of:
capturing a sequence of images with the image capturing device such that a field of view of the image capturing device captures images of at least one of a plurality of pedicle markers placed on a plurality of guide wires, respectively, or a plurality of guide wires, the plurality of pedicle markers or the plurality of guide wires arranged at a surgical incision of a body of a living being undergoing orthopedic surgery;
providing for a live video feed on the display device by either displaying at least some of images of the captured images or by a direct view with a transparent display device;
detecting the plurality of pedicle markers or the plurality of guide wires with the data processing device based on the captured sequence of images;
first calculating an orientation and position of the detected plurality of pedicle markers or the detected plurality of guide wires; and
second calculating pose data information for at least two vertebrae based on the orientation and the position of at least one of the detected plurality of pedicle markers or the detected plurality of guide wires that are attached to the vertebra from the step of first calculating.

16. The method of claim 15, further comprising the steps of:
third calculating parameters that characterize the spinal column from the pose data information from the step of second calculating; and
displaying the parameters that characterize the spinal column on the display device; or
further comprising the step of
fourth calculating a virtual three-dimensional (3D) position of a position of a screw head of each pedicle screw based on the orientation and the position of the first calculating; and
projecting and displaying each calculated virtual 3D position of the plurality of screw heads with a graphical element with a graphical user interface on the display device at a location that corresponds to the location of the screw head projected to a currently provided image of the live video feed.
